# EUROPÄISCHE PATENTSCHRIFT

(11) **EP 2 846 971 B1**
(45) Veröffentlichungstag und Bekanntmachung des Hinweises auf die Patenterteilung: **18.12.2019**
(21) Anmeldenummer: 13720414.5
(22) Anmeldetag: 06.05.2013
(51) Int. Cl.: B25J 17/02, G09B 9/00, G09B 9/12, G09B 9/30

(54) **MANIPULATORANORDNUNG UND BEWEGUNGSVORRICHTUNG**
MANIPULATOR ARRANGEMENT AND MOVEMENT DEVICE
SYSTÈME MANIPULATEUR ET DISPOSITIF DE DÉPLACEMENT

(30) Priorität: 08.05.2012 AT 5472012
(43) Veröffentlichungstag der Anmeldung: 18.03.2015
(73) Patentinhaber: AMST-Systemtechnik GmbH, 5282 Ranshofen (AT)
(72) Erfinder: SCHLÜSSELBERGER, Richard sen., A-5280 Braunau am Inn (AT); SCHLÜSSELBERGER, Richard jun., A-5280 Braunau am Inn (AT); SCHLÜSSELBERGER,Rainer, A-5280 Braunau am Inn (AT); EISENKÖCK, Norman, A-4710 Grieskirchen (AT)
(74) Vertreter: Puchberger & Partner Patentanwälte
(86) Internationale Anmeldenummer: PCT/EP2013/059342
(87) Internationale Veröffentlichungsnummer: WO 2013/167511

(56) Entgegenhaltungen:
- US-A- 5 051 094
- US-A1- 2011 126 660
- Jaeheung Park: "CONTROL STRATEGIES FOR ROBOTS IN CONTACT", , 1 March 2006 (2006-03-01), XP055361767, Retrieved from the Internet: URL:https://cs.stanford.edu/group/manips/p ublications/pdfs/Park_2006_thesis.pdf [retrieved on 2017-04-04]
- Ole Jakob Elle: "Sensor Control in Robotic Surgery", , 1 August 2003 (2003-08-01), pages 1-264, XP055362381, University of Oslo Retrieved from the Internet: URL:http://citeseerx.ist.psu.edu/viewdoc/d ownload?doi=10.1.1.385.3413&rep=rep1&type= pdf [retrieved on 2017-04-06]
- PAL J FROM ET AL: "Modeling and motion planning for mechanisms on a non-inertial base", 2009 IEEE INTERNATIONAL CONFERENCE ON ROBOTICS AND AUTOMATION : (ICRA) ; KOBE, JAPAN, 12 - 17 MAY 2009, IEEE, PISCATAWAY, NJ, USA, 12 May 2009 (2009-05-12), pages 3320-3326, XP031509900, ISBN: 978-1-4244-2788-8
- W.-H. Zhu ET AL: "Motion/force/image control of a diagnostic ultrasound robot", Robotics and Automation, 2000. Proceedings. ICRA '00. IEEE International Conference on, vol. 2, 1 January 2000 (2000-01-01), pages 1580-1585, XP055525497, Piscataway, NJ, USA DOI: 10.1109/ROBOT.2000.844822 ISBN: 978-0-7803-5886-7
- RYOHEI TAKEUCHI ET AL: "Field Testing of a Remote Controlled Robotic Tele-echo System in an Ambulance Using Broadband Mobile Communication Technology", JOURNAL OF MEDICAL SYSTEMS, KLUWER ACADEMIC PUBLISHERS-PLENUM PUBLISHERS, NE, vol. 32, no. 3, 29 January 2008 (2008-01-29), pages 235-242, XP019609191, ISSN: 1573-689X

## Beschreibung

Die Erfindung betrifft eine Manipulatoranordnung zur berührenden und/oder invasiven Untersuchung oder Behandlung am menschlichen oder tierischen Körper unter dem Einfluss erhöhter und/oder wechselnder Beschleunigung und eine Bewegungsvorrichtung mit zumindest einer Befestigungsvorrichtung zur Aufnahme und/oder Befestigung eines menschlichen oder tierischen Körpers, die an einem ersten Trägerelement vorgesehen ist, das um eine erste Hauptachse drehbar angeordnet ist sowie die Verwendung einer Manipulatoranordnung in einer Bewegungsvorrichtung.

Zur Ausbildung oder zum Training von Piloten oder zur Vorbereitung von Personen auf erhöhte, wechselnde Beschleunigungszustände sind Vorrichtungen bekannt, bei denen eine Befestigungsvorrichtung zur Aufnahme und/oder Befestigung einer Person um eine erste Hauptachse drehbar angeordnet ist. Durch die Drehung in einem gewissen Normalabstand zur Hauptdrehachse erfährt die Person eine erhöhte Beschleunigung. Die Größe der Beschleunigung setzt sich dabei aus der Radialbeschleunigung und der Erdbeschleunigung zusammen. Die Größe des Vektors kann somit durch Änderung der Winkelgeschwindigkeit um die erste Hauptachse und/oder durch Änderung des Normalabstandes variiert werden.

Ferner können weitere Freiheitsgrade und Antriebsanordnungen vorgesehen sein, um den menschlichen oder tierischen Körper in der Bewegungsvorrichtung relativ zum resultierenden Beschleunigungsvektor zu positionieren. Durch diese Positionierung kann somit die Lage des resultierenden Beschleunigungsvektors zur Körperlage der Person gewählt werden.

Beispiele für derartige Vorrichtungen sind Flugsimulatoren, Einarmzentrifugen, Mehrarmzentrifugen mit verfahrbarem Schlitten, Mehrarmzentrifugen mit verfahrbarem Heaveschlitten, medizinische Zentrifugen mit mehreren um eine Hauptachse drehbar angeordneten Nacellen etc. Beispielsweise zeigen die EP2351001 A1 und die US 5,051,094 A gattungsähnliche Vorrichtungen. Aus der angeführten US 5,051,094 A ist eine Zentrifuge bekannt, die zur Erzeugung dauerhaft erhöhter und/oder wechselnder Beschleunigungen eingerichtet ist. Herkömmliche Vorrichtungen aus anderen technischen Gebieten, wie beispielsweise Roboterarme aus dem Gebiet der Robotik, sind beispielsweise aus der US 2011/126660 A1 und dem Artikel "Field Testing of a Remote Controlled Robotic Tele-echo System in an Ambulance Using Broadband Mobile Communication Technology" des Journals of Medical Systems, Bd. 32, Nr. 3, 29. Jänner 2008, Seite 235-242, XP019609191 (ISSN: 1573-689X) bekannt. Aus der angeführten US 2011/126660 A1 ist ein passiver Sicherheitsmechanismus für einen Roboterarm bekannt, dessen Grundprinzip auf einer Parallelführung mit einer Drehmomentbegrenzung basiert. Aus dem angeführten Artikel ist ein ferngesteuertes Roboter-Tele-Echo-System bekannt, welches Metallstangen mit Dehnungsmessstreifen verwendet, um eine übermäßige Gewaltanwendung auf den Probanden zu verhindern.

Zur Überwachung der Körperfunktionen der Insassen derartiger Bewegungsvorrichtungen können gemäß dem Stand der Technik Parameter wie Puls oder auch die Atemfrequenz gemessen werden. Jedoch sind weitere Messungen wie beispielsweise Ultraschallaufnahmen von Organen, Blutuntersuchungen oder ähnliches in Systemen erhöhter oder wechselnder Beschleunigung nicht möglich. Grund dafür ist, dass durch die Änderung der Beschleunigung auch die Organe des Körpers eine Bewegung im Körper erfahren und zumindest teilweise die Lage ändern. Ferner werden gegebenenfalls auch Teile des Körpers oder der gesamte Körper durch den Einfluss erhöhter Beschleunigung bewegt. Bei statisch fix am Körper montierten Messinstrumenten kann es somit vorkommen, dass die Messergebnisse durch die beschriebenen Änderungen im Körper verfälscht werden und somit nicht aussagekräftig sind. Zur Verbesserung der Untersuchung oder der Behandlung ist es somit notwendig, eine Justiermöglichkeit des Messinstruments vorzusehen. Da jedoch in Systemen erhöhter oder wechselnder Beschleunigung eine Behandlung oder Untersuchung durch beispielsweise medizinisches Personal nicht möglich ist, müssten die Handlungen durch Handhabungsvorrichtungen oder Roboter durchgeführt werden. Ferner ist auch bei Verwendung einer Strahlenquelle am Funktionskopf, z.B. für Röntgenaufnahmen, eine ferngesteuerte Bedienung zum Schutz der Bedienperson von Vorteil.

Dem Stand der Technik entsprechende Roboter oder Handhabungsgeräte sind jedoch nicht dazu geeignet, den Belastungen in beispielsweise einer Zentrifuge oder ähnlichen Bewegungsvorrichtungen Stand zu halten. Ferner umfassen dem Stand der Technik entsprechende Roboter oder Manipulatoranordnungen Steuerungen, die nicht dazu eingerichtet und ausgelegt sind, erhöhte oder wechselnde Beschleunigungen zu berücksichtigen. Darüber hinaus besteht bei Untersuchungen von Testpersonen durch Roboter ein erhöhtes Risiko, dass es bei Fehlfunktionen des Roboters zu Verletzungen kommt.

Aufgabe der Erfindung ist es nun, eine Bewegungsvorrichtung und eine Manipulatoranordnung zu schaffen, die dazu geeignet und eingerichtet ist eine Untersuchung oder Behandlung am menschlichen oder tierischen Körper bei erhöhter oder wechselnder Beschleunigung zu ermöglichen. Die Aufgabe der Erfindung wird durch die Merkmale des unabhängigen Anspruchs gelöst. Die Aufgabe umfasst gegebenenfalls die Teilaufgaben, dass die Untersuchung ferngesteuert, von einer Position außerhalb der Bewegungsvorrichtung durchführbar ist, dass die Untersuchung durch eine Manipulatoranordnung durchgeführt wird, die dazu ausgelegt ist, in Systemen wechselnder oder erhöhter Beschleunigung eingesetzt zu werden und dass eine ungewollte Verletzung des Körpers durch die Manipulatoranordnung ausgeschlossen ist.

Die erfindungsgemäße Aufgabe wird dadurch gelöst, dass ein Funktionskopf relativ zu einer Basis entlang mehrerer, durch Antriebsvorrichtungen bewegbarer Freiheitsgrade bewegbar ist und dass zumindest eine Antriebsvorrichtung als kraftbegrenzte Antriebsvorrichtung ausgeführt ist.
Dazu kann vorgesehen sein, dass die Kontaktkraft zwischen dem Funktionskopf und dem Körper durch die kraftbegrenzte Antriebsvorrichtung systeminhärent begrenzt ist, insbesondere, dass die Kontaktkraft über ein Druckventil der kraftbegrenzten Antriebsvorrichtung begrenzt ist, dass die Kontaktkraft wählbar ist und/oder dass das Druckventil als Regelventil ausgeführt ist. Weiters kann die erfindungsgemäße Vorrichtung die Merkmale umfassen, dass bei Überschreiten der gewählten maximalen Kontaktkraft das Bewegungselement der kraftbegrenzten Antriebsvorrichtung vom Basiselement der kraftbegrenzten Antriebsvorrichtung im Wesentlichen entkoppelt ist, bevorzugt in Wirkrichtung entkoppelt ist. Die Entkoppelung geschieht beispielsweise über die Öffnung des Überdruckventils.

Ferner umfasst die erfindungsgemäße Vorrichtung bevorzugt die Merkmale, dass die Antriebsvorrichtungen von einer oder mehreren Steuerungseinrichtungen gesteuert und/oder geregelt sind und dass die Antriebsvorrichtungen und die Steuerungseinrichtungen zum Betrieb bei erhöhter und/oder wechselnder Beschleunigung eingerichtet sind, dass die Lage und/oder die Kontaktkraft des den Körper berührenden Funktionskopfs gegenüber dem Körper unter dem Einfluss erhöhter und/oder wechselnder Beschleunigung veränderbar ist und/oder dass der Funktionskopf in wählbarer Stellung an den Körper führbar ist

Weitere erfindungsgemäße Merkmale können sein, dass die kraftbegrenzte Antriebsvorrichtung eine oder mehrere kolbenlose pneumatische Aktoren, eine oder mehrere Luftmuskelanordnungen, eine oder mehrere Luftbalganordnungen, eine oder mehrere Pneumatikzylinderanordnungen mit Pneumatikzylindern mit im Wesentlichen haftreibungsfrei gelagerten Kolben, eine oder mehrere getriebelose elektrische Lineareinheiten mit im Wesentlichen haftreibungsfrei gelagerten Ankern und/oder eine oder mehrere Führungen umfasst, dass die Antriebsvorrichtungen eine Linearachse, einen Zahnstangenantrieb, einen Parallelkinematikantrieb, einen Hexapod, einen Tripod, einen Roboterarm, einen Rotationsantrieb, einen kardanischen Antrieb und/oder einen kartesischen Antrieb umfassen und/oder dass die Antriebsvorrichtungen jeweils ein Basiselement, ein Bewegungselement und einen Antrieb zur Bewegung des Bewegungselements gegenüber dem Basiselement entlang des oder der jeweiligen Freiheitsgrade umfassen und/oder dass die Antriebsvorrichtungen seriell aneinandergereiht sind, wobei jeweils das Bewegungselement einer Antriebsvorrichtung mit dem Basiselement der darauffolgenden Antriebsvorrichtung verbunden oder gekoppelt ist.

Ferner kann vorgesehen sein, dass die Bewegung des Funktionskopfes gegenüber der Basis fernsteuerbar und/oder automatisierbar ist, insbesondere über eine oder mehrere Steuerungseinrichtungen und/oder eine oder mehrere Dateneingabeanordnungen fernsteuerbar und/oder automatisierbar ist, dass die Dateneingabeanordnung Eingabegeräte wie Joysticks, Schieberegler, Datenhandschuhe, Computerprogramme, automatisierte Programme und/oder ähnliche Anordnungen umfasst, dass der Funktionskopf Komponenten, Untersuchungs- und/oder Behandlungsvorrichtungen wie beispielsweise einen Ultraschallmesskopf, optische Aufnahmegeräte, akustische Aufnahmegeräte, Widerstandsmessgeräte, eine Injektionsanordnung, eine Flüssigkeitsanalyseanordnung, eine Blutabnahmevorrichtung, eine Analysevorrichtung, eine chemische Analysevorrichtung, eine Strahlenquelle z.B. Röntgen-, Gamma- oder Infrarotstrahlung, eine Laserquelle, Probenentnahmevorrichtungen, Temperaturmessgeräte, Strommessgeräte, Strahlendetektionsvorrichtungen, endoskopische Untersuchungsgeräte, Vorrichtungen zur optischen Augenuntersuchung und/oder weitere radiologische, invasive oder berührende Vorrichtungen zu diagnostischen oder therapeutischen Zwecken umfasst.
Merkmale der Erfindung sind gegebenefalls auch, dass eine erfindungsgemäße Manipulatoranordnung an einer erfindungsgemäßen Bewegungsvorrichtung angeordnet ist, dass die Bewegungsvorrichtung als Flugsimulator, als Einarmzentrifuge, als Zentrifuge mit verfahrbarem Schlitten, als Zentrifuge mit verfahrbarem Heaveschlitten, als Trainingszentrifuge, als Trainingszentrifuge zum Einsatz unter Schwerelosigkeit, als medizinische Zentrifuge oder als medizinische Zentrifuge mit mehreren um eine erste Drehachse drehbar angeordneten Nacellen ausgeführt ist, dass ein mit dem ersten Trägerelement gekoppeltes oder verbundenes Basisträgerelement vorgesehen ist, das mit der Basis der Manipulatoranordnung verbunden, gekoppelt oder verbindbar ist und/oder dass der Funktionskopf über die kraftbegrenzte Antriebsvorrichtung oder über die kraftbegrenzten Antriebsvorrichtungen im Wesentlichen entlang einer Tangentialebene der Hauptachse bewegbar ist.

Weitere Merkmale sind, dass der Funktionskopf während der Drehung um die Hauptachse über die Betätigung der Manipulatoranordnung durch eine Dateneingabeanordnung ferngesteuert oder automatisiert an den Körper führbar und/oder zum Körper positionierbar ist, dass der Funktionskopf während der Drehung um die Hauptachse über die Betätigung der Manipulatoranordnung durch eine Dateneingabeanordnung ferngesteuert oder automatisiert in wählbarer Stellung und/oder mit wählbarer Kontaktkraft an den Körper führbar, andrückbar und/oder zum Körper positionierbar ist, dass die Stellung und/oder die Kontaktkraft zwischen dem Funktionskopf und dem Körper während der Drehung um die Hauptachse über die Betätigung der Manipulatoranordnung durch eine Dateneingabeanordnung ferngesteuert oder automatisiert veränderbar ist und/oder dass die Kontaktkraft systeminhärent begrenzt ist, insbesondere, dass die Kontaktkraft über ein Druckventil der kraftbegrenzten Antriebsvorrichtung begrenzt ist, wobei die Kontaktkraft wählbar ist und wobei das Druckventil als Regelventil ausgeführt ist.

Bei Überschreiten der maximalen Kontaktkraft ist das Bewegungselement der kraftbegrenzten Antriebsvorrichtung vom Basiselement der kraftbegrenzten Antriebsvorrichtung im Wesentlichen entkoppelt, bevorzugt in Wirkrichtung entkoppelt.

Erfindungsgemäß ist eine Manipulatoranordnung vorgesehen, die unter dem Einfluss erhöhter und/oder wechselnder Beschleunigung einen Funktionskopf an den Körper führen kann. Dieser Funktionskopf kann Komponenten, Untersuchungs- und/oder Behandlungsvorrichtungen wie z.B. einen Ultraschallmesskopf, optische Aufnahmegeräte, akustische Aufnahmegeräte, Widerstandsmessgeräte, eine Injektionsanordnung, eine Flüssigkeitsanalyseanordnung, eine Blutabnahmevorrichtung, eine Analysevorrichtung, eine chemische Analysevorrichtung, eine Strahlenquelle z.B. Röntgen-, Gamma- oder Infrarotstrahlung, eine Laserquelle, Probenentnahmevorrichtungen, Temperaturmessgeräte, Strommessgeräte, Strahlendetektionsvorrichtungen, endoskopische Untersuchungsgeräte, Vorrichtungen zur optischen Augenuntersuchung und/oder weitere radiologische, invasive oder berührende Vorrichtungen zu diagnostischen oder therapeutischen Zwecken umfassen.

Durch die erfindungsgemäße Anordnung können beispielsweise Aufgaben wie Ultraschallaufnahmen von Organen und/oder Blutgefäßen, Blutuntersuchungen, Abhören der Herz- und/oder Lungenfunktion, Hautwiderstandsmessungen, Anfertigen von Röntgenbildern, Bestrahlung, Erwärmung z.B. zur Erhöhung der Durchblutung, Durchblutungsmessung im tieferen Gewebe, Körperfettmessungen, Gehirnstrommessungen, kardiologische Messungen und weitere radiologische, invasive oder berührende Aufgaben zu diagnostischen oder therapeutischen Zwecken durchgeführt werden.

Die Manipulatoranordnung umfasst dabei eine oder mehrere Antriebsvorrichtungen, die die Bewegung des Funktionskopfs gegenüber einer Basis ermöglichen. Die Manipulatoranordnung ist derart ausgeführt, dass eine unbeabsichtigte Verletzung des Körpers durch den Funktionskopf und/oder die Manipulatoranordnung ausgeschlossen ist. Zu diesem Zweck ist in bevorzugter Weise ein systeminhärenter Sicherheitsmechanismus vorgesehen, der die Kraft, die durch die Manipulatoranordnung auf den Körper ausgeübt wird, begrenzt. Dazu ist zumindest eine Antriebsvorrichtung der Manipulatoranordnung als nachgiebige oder sensitive Antriebsvorrichtung ausgebildet. Beispiele für derartige kraftbegrenzte Antriebsvorrichtungen sind z.B. Anordnungen, die im Wesentlichen haftreibungsbefreite Stellelemente wie Luftmuskel, pneumatische Stellelemente, Luftbalge etc. umfassen. Bei derartigen Vorrichtungen ist ein Bewegungselement gegenüber einem Basiselement durch Veränderung der Länge eines elastischen Elements wie beispielsweise einem Luftmuskel oder einem Luftbalg bewegbar. Zusätzlich kann die Vorrichtung einen Wegsensor sowie ein gegen den Luftmuskel oder den Balg wirkendes elastisches Element, wie z.B. eine Feder umfassen. Neben dem Wegsensor ist bevorzugt auch ein Kraftsensor vorgesehen, der zur Steuerung und zur Begrenzung der, durch die Antriebsvorrichtung ausgeübten Kraft dient. In bevorzugter Weise ist das Stellelement pneumatisch durch Gasdruck, insbesondere durch Luftdruck betätigbar. Dazu wird von einem Kompressor verdichtetes Gas in den elastischen Körper geleitet. Diese Einleitung des Drucks in den elastischen Körper ist über ein Regelventil geregelt und/oder gesteuert. Zur systeminhärenten Begrenzung der Kraft kann der Gasdruck derart über ein Überdruckventil wirken, dass bei Erreichen der gewünschten Maximalkraft durch das Bewegungselement auf beispielsweise einen Körper das Überdruckventil öffnet und somit den Druck und die Kraft begrenzt. Der Druck ist über die druckbeaufschlagte Fläche direkt proportional zur ausgeübten Kraft.

Beispiele für kraftbegrenzte Antriebsvorrichtungen sind:
- Anordnungen kolbenloser, pneumatischer Aktoren wie die genannten Luftbalganordnungen mit einem Balgzylinder oder Luftmuskelanordnungen mit einem Luftmuskel,
- Luftbalganordnungen mit einem Balgzylinder mit entgegenwirkender Spiralfeder,
- Luftmuskelanordnungen mit gegenwirkender Feder,
- die Anordnung mehrerer Balgzylinder oder Luftmuskel,
- Pneumatikzylinderanordnungen mit einem Pneumatikzylinder mit haftreibungsfrei gelagertem Kolben wie beispielsweise Gaszylinder mit Graphitkolben,
- getriebelose elektrische Lineareinheiten mit haftreibungsfrei gelagertem, luft- oder magnetgelagertem Anker,
- die Anordnung mehrerer Aktoren, wie Pneumatikzylinder Luftmuskelanordnungen und/oder Balganordnungen als Parallelkinematikanordnung
- Anordnungen von pneumatischen Aktoren mit z.B. pneumatischen Federn, elastischen Elementen, Spiralfedern, Führungen,
- Kombinationen einer oder mehrerer der obengenannten Anordnungen,
- etc.

Die kraftbegrenzte Antriebsvorrichtung, insbesondere eine kraftbegrenzte Antriebsvorrichtung mit einem oder mehreren der obengenannten pneumatischen Aktoren kann gegebenenfalls eine oder mehrere Führungen umfassen. Diese Führungen dienen der Stabilisierung der Bewegung des Bewegungselements entlang des jeweiligen Freiheitsgrades. Auch diese Führungen sind dazu geeignet und/oder eingerichtet unter dem Einfluss erhöhter und/oder wechselnder Beschleunigung eigesetzt zu werden. Beispiele für derartige Führungen sind Linearführungen, Rotationsführungen und insbesondere Führungen, die im Wesentlichen haftreibungsbefreit sind.

Zur Begrenzung der Kraft ist neben der Regelung der Aktoren auf die Reibung und die Trägheit der Bewegung des Bewegungselements zu achten. Insbesondere Stellglieder, die im Wesentlichen haftreibungsbefreit sind, sind zum Einsatz in der erfindungsgemäßen Manipulatoranordnung als kraftbegrenzte Antriebsvorrichtung geeignet.

Die Steuerung der Bewegung des Funktionskopfes, insbesondere die Steuerung der Antriebe geschieht in bevorzugter Weise durch eine Steuerungseinrichtung. Diese ist mit den einzelnen Antrieben verbunden und dazu eingerichtet, diese zu steuern oder zu regeln. Dazu kann jede Achse der Antriebsvorrichtungen einzeln angesteuert und steuerbar sein oder eine Multiachssteuerung vorgesehen sein. Bei der einzelnen Ansteuerung der Achsen kann somit jeder lineare Freiheitsgrad getrennt gesteuert werden. Ferner kann jeder Rotationsfreiheitsgrad einzeln gesteuert sein.

Bei einer Multiachssteuerung werden über eine oder mehrere Dateneingabeanordnungen mehrere Achsen gleichzeitig gesteuert. Durch Transformation des Steuerungskoordinatensystems auf einen beliebigen Punkt kann die Bewegungscharakteristik der Manipulatoranordnung angepasst werden. Bevorzugt kann der Steuerungspunkt in einen Berührpunkt des Funktionskopfs mit dem Körper gelegt werden.

Darüber hinaus kann eine Dateneingabeanordnung vorgesehen sein. Diese dient im Wesentlichen der Eingabe von Anweisungen an die Steuerungseinrichtung die z.B. zur Ausführung einer Bewegung der Antriebe der Manipulatoranordnung weitergegeben werden. Derartige Dateneingabeanordnungen können beispielsweise als Joysticks, Schieberegler, Schieber, virtuelle Datenhandschuhe, optische Aufnahmegeräte oder auch durch einen Computer und/oder ein Computerprogramm ausgeführt sein. Die Dateneingabeanordnungen können in bevorzugter Weise außerhalb der Bewegungsvorrichtung, beispielsweise in einem Kontrollraum angeordnet sein. Über die Dateneingabeanordnungen kann der Funktionskopf ferngesteuert oder automatisiert an den Körper geführt werden, um Untersuchungen und/oder Behandlungen vorzunehmen.

Zur Verbesserung der Bedienbarkeit können auch Dateneingabeanordnungen mit steuer- oder regelbarer Verstellkraft vorgesehen sein. Diese unter dem Begriff "force feedback" bekannten Eingabegeräte übertragen Signale der von der Manipulatoranordnung oder dem Funktionskopf aufgenommenen Kräfte auf die Dateneingabeanordnungen. Dadurch entsteht bei der Bedienung der Eindruck des direkten "fühlenden" Kontaktes. Ferner kann ein optisches Aufnahmegerät wie beispielsweise eine Kamera vorgesehen sein, die ein Videosignal an einen Monitor leitet. Über diesen Monitor ist es für die Bedienperson möglich, die Untersuchung auch optisch überblicken zu können. Das optische Aufnahmegerät ist bevorzugt mit dem Basisträgerelement, mit der Basis der Manipulatoranordnung und/oder mit der Befestigungsvorrichtung verbunden. Der Monitor befindet sich in bevorzugter Weise im Bereich der Dateneingabeanordnung.

Als Manipulatoranordnung wird eine Vorrichtung definiert, die einen Funktionskopf relativ zu einer Basis entlang einer oder mehrerer Freiheitsgrade bewegen kann. Die Manipulatoranordnung ist dabei steuer- und/oder regelbar und bevorzugt fernsteuerbar. Die Manipulatoranordnung kann unterschiedliche Antriebsvorrichtungen umfassen, die seriell oder parallel aneinandergereiht sind. Als serielle Aneinanderreihung von Antriebsvorrichtungen wird eine Aneinanderreihung definiert, bei der an den bewegten Komponenten einer ersten Antriebsvorrichtung eine zweite Antriebsvorrichtung vorgesehen ist. Somit ist die Bewegung der zweiten Antriebsvorrichtung von der Bewegung der ersten Antriebsvorrichtung abhängig. Die Bewegung der ersten Antriebsvorrichtung ist jedoch unabhängig von der Bewegung der zweiten Antriebsvorrichtung. Das parallele Vorsehen von Antriebsvorrichtungen entspricht beispielsweise einer Parallelkinematikanordnung. Bei Parallelkinematikanordnungen sind die Freiheitgrade im kinematischen Sinn zwar entkoppelt, bei Multiachssteuerungen kann jedoch durch die Steuerung ein Koppelung gegeben sein. Die erfindungsgemäße Manipulatoranordnung ist dazu eingerichtet und geeignet, in Systemen erhöhter und/oder wechselnder Beschleunigung eingesetzt zu werden.

Als erhöhte und/oder wechselnde Beschleunigung ist ein Beschleunigungszustand definiert, bei dem die Manipulatoranordnung erhöhte oder wechselnde Beschleunigungskräfte erfährt. Insbesondere bedeutet dies, dass die Manipulatoranordnung selbst bewegt wird. Durch diese Bewegung, insbesondere durch die Änderung der Bewegung wirken auf die Manipulatoranordnung und auf den Funktionskopf Beschleunigungskräfte, die von den Beschleunigungskräften der Umgebung, z.B. den Erdbeschleunigungskräften, abweichen. Im schwerelosen Raum gelten Beschleunigungen größer null als erhöht, da die Beschleunigung der Umgebung im Wesentlichen gleich null ist. Bevorzugt gilt als erhöhte und/oder wechselnde Beschleunigung ein Zustand dauerhaft erhöhter Beschleunigung, wie er beispielsweise in einer Zentrifuge auftritt. Unter dem Einfluss der Erdbeschleunigung wäre dies beispielsweise eine erhöhte Beschleunigung von etwa 1,2G bis 6G - also 1,2- bis sechsfacher Erdbeschleunigung. In erfindungsgemäßen Bewegungsvorrichtungen, wie beispielsweise in einer Zentrifuge, können jedoch auch erhöhte Beschleunigungen von bis zu 15G und mehr auftreten. Im schwerelosen Raum entspräche eine erhöhte Beschleunigung beispielsweise Beschleunigungen von etwa 0,1G bis 6G und darüber. In bevorzugter Weise wird die Manipulatoranordnung entlang einer Kreisbahn um eine Hauptachse gedreht. Durch die Drehung mit einem gewissen Normalabstand zur Hauptachse ist die Beschleunigung gegenüber der Umgebung erhöht.

Die kraftbegrenzte Antriebsvorrichtung ist in bevorzugter Weise entlang einer Tangentialebene der Hauptachse bewegbar. Als Tangentialebene ist eine Ebene definiert, die im Wesentlichen der Normalebene auf einen Radialvektor durch die Hauptachse entspricht. Bevorzugt steht der Radialvektor orthogonal zur Hauptachse. Eine Tangentialebene ist somit bevorzugt eine Tangentialebene einer Kreisbahn um die Hauptachse, wobei die Tangentialebene bevorzugt auch parallel zur Hauptachse verläuft. Die Richtung des Radialvektors entspricht im Wesentlichen der Richtung des auf den Körper oder auf die Manipulatoranordnung wirkenden Beschleunigungsvektors oder der Richtung jenes Beschleunigungsvektors, der durch die Bewegungsvorrichtung erzeugt wird. Bei rein linear verfahrbaren Schlitten beispielsweise ist als Tangentialebene die Normalebene des erzeugten Beschleunigungsvektors definiert.

Die Wirkrichtung der kraftbegrenzten Antriebsvorrichtung kann beispielsweise zur Ultraschalluntersuchung normal auf die Oberfläche des Körpers stehen oder auch zwischen 30° und 50° zu dieser schräg gestellt sein.

Als Funktionskopf ist eine Vorrichtung bezeichnet, die unterschiedliche Werkzeuge, Sensoren, Aufnahmegeräte oder Analysegeräte umfassen kann. Dabei können mehrere dieser Komponenten vorgesehen sein, oder auch nur eine.

Gemäß der vorliegenden Erfindung kann der Funktionskopf durch die Manipulatoranordnung in unterschiedlichen Stellungen und mit unterschiedlichen Kontaktkräften an den Körper und entlang des Körpers geführt werden. Weiters kann die Stellung des Funktionskopfes gegenüber dem Körper bei erhöhter und/oder wechselnder Beschleunigung verändert werden. Bevorzugt weist die Manipulatoranordnung sechs Freiheitsgrade auf. Die Bewegung der Antriebsvorrichtungen geschieht in bevorzugter Weise positionsgesteuert. Die Bewegung der kraftbegrenzten Antriebsvorrichtung bevorzugt kraftgesteuert.

Weiters kann die Kontaktkraft bzw. die Andrückkraft des Funktionskopfs an den Körper durch die erfindungsgemäße Manipulatoranordnung und die kraftbegrenzte Antriebsvorrichtung auf einem konstanten oder bestimmten Wert gehalten werden. Der Wert kann z.B. über eine Dateneingabeanordnung ferngesteuert eingegeben werden oder automatisiert von einem Programm vorgegeben sein. Die Kontakt- oder Andrückkraft ist dabei bevorzugt unabhängig oder entkoppelt von der Bewegung der Bewegungsvorrichtung und der auf den Körper und/oder die Manipulatoranordnung wirkenden Beschleunigung. Die Kontaktkraft kann über die Dateneingabeanordnung verändert, gewählt und/oder begrenzt werden. Zur Steuerung der Bewegung, insbesondere zur Rotation des Funktionskopfs kann vorgesehen sein, dass der Drehpunkt des Funktionskopfs in dem Berührpunkt des Funktionskopfs mit dem Körper liegt. Die Steuerung kann dabei gegebenenfalls durch Steuerung der Linearantriebe unterstützt sein und als Multiachs-Steuerung ausgeführt sein.

Erfindungsgemäß ist die Manipulatoranordnung gegebenenfalls an einer Bewegungsvorrichtung vorgesehen. Beispiele für Bewegungsvorrichtungen sind Flugsimulatoren, Zentrifugen, Zentrifugen mit verfahrbarem Schlitten, linear verfahrbare Schlitten, medizinische Zentrifugen, Trainingszentrifugen etc. Praktische Anwendungen der Manipulatoranordnungen in Kombination mit einer Bewegungsvorrichtung ist beispielsweise die Untersuchung von Personen in Einarmzentrifugen oder die Untersuchung von Personen in linearen verfahrbaren Vorrichtungen beispielsweise zur Längsdynamiksimulation. Weiters kann die erfindungsgemäße Bewegungsvorrichtung im schwerelosen Raum, beispielsweise in Raumstationen oder Raumschiffen vorgesehen sein. Insbesondere bei langem Aufenthalt von Personen oder Tieren im All ist eine Simulation von Beschleunigungen, beispielsweise durch eine Zentrifuge notwendig, um einen muskulären Abbau sowie die Schwächung des Skeletts zu verhindern. Dazu sind beispielsweise eine oder mehrere Nacellen drehbar um eine Hauptachse angeordnet. Durch Drehung der einzelnen Nacellen in einem gewissen Normalabstand erfahren die auf den Nazellen positionierten Personen eine gewisse Beschleunigung. Durch das Vorsehen von Trainingsgeräten wie beispielsweise einem Ergometer können somit im schwerelosen Raum Trainingseinheiten unter dem Einfluss normaler, wechselnder oder erhöhter Beschleunigung durchgeführt werden. Derartige medizinische Zentrifugen oder auch Trainingszentrifugen können insbesondere zu wissenschaftlichen Zwecken auch auf der Erde, unter dem Einfluss der Erdbeschleunigung eingesetzt sein. Auch diese Bewegungsvorrichtungen entsprechen einer Anwendung der vorliegenden Erfindung.

In weiterer Folge wird die Erfindung anhand konkreter, schematisch dargestellter Ausführungsbeispiele weiter beschrieben.
Fig. 1 zeigt eine schematische Schrägansicht einer erfindungsgemäßen Bewegungsvorrichtung umfassend eine erfindungsgemäße Manipulatoranordnung.
Fig. 2 zeigt eine mögliche Ausführungsform einer Manipulatoranordnung.
Fig. 3 zeigt eine weitere mögliche Ausführung der Manipulatoranordnung, insbesondere ein Detail der kraftbegrenzten Antriebsvorrichtung.
Fig. 4 zeigt eine weitere Ausführungsform einer Manipulatoranordnung mit einer kraftbegrenzten Antriebsvorrichtung.
Fig. 5 zeigt eine mögliche Manipulatoranordnung mit Teilen einer Bewegungsvorrichtung in einer Schrägansicht.
Fig. 6 zeigt dieselbe Anordnung wie Fig. 5, jedoch in einer weiteren Ansicht.
Fig. 7 zeigt eine weitere Ausführungsform einer möglichen Manipulatoranordnung mit Teilen einer Bewegungsvorrichtung in einer schematischen Schrägansicht.
Fig. 8 zeigt dieselbe Anordnung wie Fig. 7, jedoch in einer weiteren Ansicht.
Fig. 9 zeigt eine weitere Ausführungsform der Manipulatoranordnung und Teilen einer Bewegungsvorrichtung.

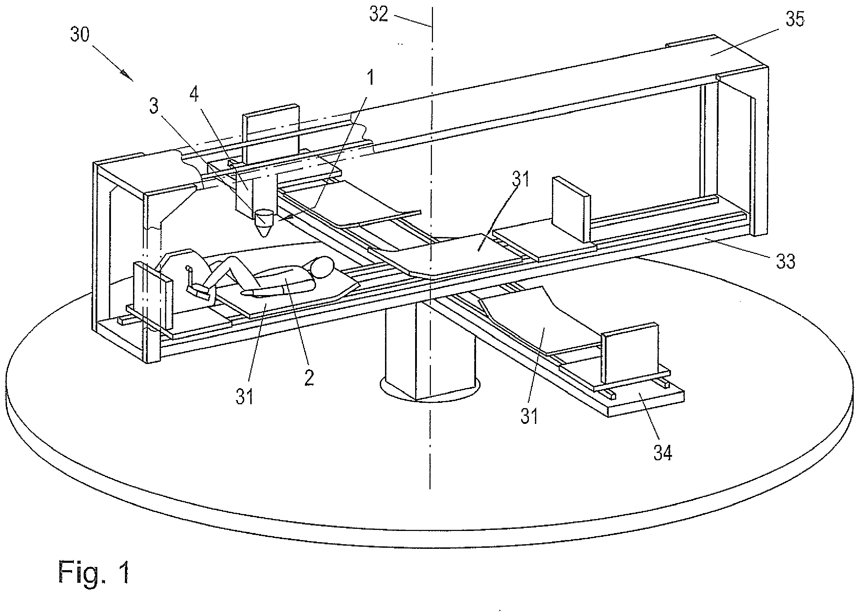

Fig. 1 zeigt eine Anordnung einer Bewegungsvorrichtung 30, umfassend mehrere Befestigungsvorrichtungen 31, die um eine Hauptachse 32 drehbar angeordnet sind. Die Anordnung entspricht einer möglichen Bewegungsvorrichtung einer medizinischen Zentrifuge oder einer Trainingszentrifuge zum Einsatz in schwerelosem Zustand oder unter dem Einfluss der Erdbeschleunigung.

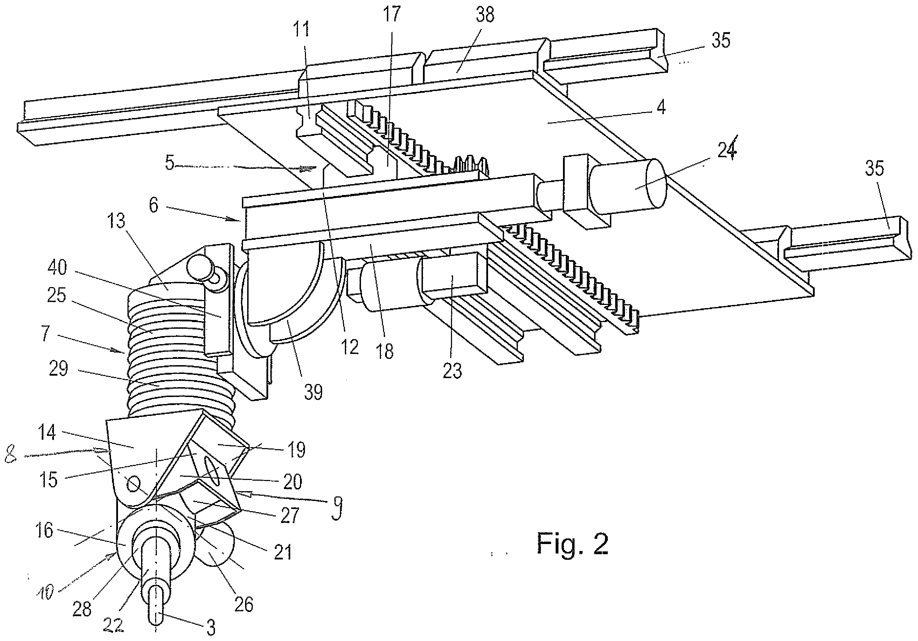

Liegend auf der Befestigungsvorrichtung 31 befindet sich ein Körper 2 einer Testperson. Die Befestigungsvorrichtung 31 ist in der vorliegenden Ausführungsform entlang eines ersten Trägerelements 33 verschiebbar angeordnet. Durch Drehung des ersten Trägerelements 33 um die Hauptachse 32 erfährt der Körper 2 eine erhöhte Beschleunigung. Diese setzt sich aus der Grundbeschleunigung, im Normalfall der Erdbeschleunigung, und der Radialbeschleunigung zusammen. Beim Einsatz in schwerelosem Zustand ist die Grundbeschleunigung im Wesentlichen Null. Die Größe der Radialbeschleunigung ist maßgeblich von der Winkelgeschwindigkeit und dem Normalabstand von der Hauptachse 32 abhängig. Um die resultierende Beschleunigung auf den Körper variieren zu können, ist in der vorliegenden Ausführung zumindest eine, bevorzugt alle Befestigungsvorrichtungen 31 verschiebbar ausgeführt, wobei die Richtung der Verschiebung im Wesentlichen radial zur Hauptachse 32 und/oder entlang des ersten bzw. des zweiten Trägerelements 33, 34 verläuft. Starr mit dem ersten Trägerelement 33 ist ein Basisträgerelement 35 verbunden. Dieses weist in der vorliegenden Ausführungsform schienenförmige Abschnitte auf, an denen die Basis 4 der Manipulatoranordnung 1 linear verschieblich angeordnet ist. In bevorzugter Weise dient diese Verschiebung der Basis 4 an dem Basisträgerelement 35 der Grobeinstellung und der Platzierung der Manipulatoranordnung 1 im Bereich des Körpers 2 der Person. An der Basis 4 der Manipulatoranordnung 1 ist über Antriebsvorrichtungen beweglich der Funktionskopf 3 angeordnet. Die Darstellung der Manipulatoranordnung 1 in Fig. 2 ist stark vereinfacht und schematisiert. Zur Durchführung von Behandlungen oder Untersuchungen am menschlichen oder tierischen Körper kann über die Steuerungseinrichtung 36 - hier nicht dargestellt - der Funktionskopf 3 der Manipulationsanordnung 1 an den Körper 2 der Person geführt werden. Dies geschieht beispielsweise über eine Dateneingabeanordnung.

Über die selbe Steuerungseinrichtung kann gegebenenfalls auch die Rotation der Bewegungsvorrichtung gesteuert sein.

Die Bewegungsvorrichtung 30 der Fig. 1 entspricht einer schematisierten Darstellung einer Vorrichtung zur medizinischen Untersuchung oder Behandlung von menschlichen oder tierischen Körpern unter dem Einfluss erhöhter oder wechselnder Beschleunigung. Dazu sind auf einem kreuzförmig angeordneten ersten und zweiten Trägerelement 33,34 mehrere Befestigungsvorrichtungen 31 vorgesehen. Auf einer derartigen Bewegungsvorrichtung können beispielsweise Leistungs- oder Belastungstests unter erhöhten Beschleunigungskräften getestet werden. Die Befestigungsvorrichtungen 31 sind in bevorzugter Weise beweglich mit dem ersten bzw. dem zweiten Trägerelement verbunden. Durch die Bewegung der Befestigungsvorrichtung mit dem Körper 2 der Person kann die resultierende Beschleunigungskraft, die auf diese Person wirkt, verringert werden, ohne den Test an den übrigen drei Befestigungsvorrichtungen zu beeinflussen. Die Bewegungsvorrichtung dreht sich in bevorzugter Weise mit konstanter Winkelgeschwindigkeit um die Hauptachse 32. Durch Wahl des Abstands des Körpers 2 von dieser Hauptachse 32 kann die resultierende Beschleunigung, die auf den Körper 2 wirkt, verändert werden.

Um unter diesen Bedingungen Untersuchungen oder Behandlungen am Körper 2 vornehmen zu können, ist der Funktionskopf 3 der Manipulatoranordnung 1 bewegbar an dem Basisträgerelement 35 vorgesehen. Über eine Steuerungseinrichtung und eine Dateneingabeanordnungen kann der Funktionskopf 3 über die Manipulatoranordnung an den Körper 2 geführt werden. Über den Funktionskopf 3 können beispielsweise Blutproben entnommen und analysiert, Ultraschallaufnahmen der Organe unter erhöhter Belastung aufgenommen oder weitere Tests am Körper durchgeführt werden.

Dazu ist es wie angemerkt zwingend notwendig, dass eine unabsichtliche Verletzung der Person 2 durch den Funktionskopf 3 ausgeschlossen ist. Zu diesem Zweck weist die erfindungsgemäße Manipulatoranordnung 1 zumindest eine kraftbegrenzte Antriebsvorrichtung auf, die einen bewegbaren Freiheitsgrad hat, der kraftbegrenzt, d.h. nachgiebig, sensitiv oder "compliant" ist.

Fig. 2 zeigt eine detailliertere Schrägansicht einer erfindungsgemäßen Manipulatoranordnung 1 und von Teilen der Bewegungsvorrichtung 30. Die Basis 4 ist schienenartig mit dem Basisträgerelement 35 verbunden. Dazu sind Führungselemente 38 der Basis 4 linear verschieblich an schienenförmigen Körpern des Basisträgerelements 35 vorgesehen. In bevorzugter Weise ist somit die Basis 4 gegenüber dem Basisträgerelement 35 verschiebbar und in gewünschter Position arretierbar angeordnet. Diese Verschiebung dient in der vorliegenden Ausführungsform zur groben Positionierung der Manipulatoranordnung 1 im Bereich des Körpers 2. An der Basis 4 ist eine erste Antriebsvorrichtung 5 vorgesehen. Diese umfasst ein erstes Basiselement 11, ein erstes Bewegungselement 17 sowie einen ersten Antrieb 23 zur Bewegung des ersten Bewegungselements 17 gegenüber dem ersten Basiselement 11. In der vorliegenden Ausführungsform ist die erste Antriebsvorrichtung im Wesentlichen als Linearachse ausgeführt. Dazu greifen Führungselemente des ersten Bewegungselements 17 in Nuten, bevorzugt in hinterschnittenen Nuten des schienenförmig ausgeführten ersten Basiselements 11 ein. Zur Bewegung ist der erste Antrieb 23 vorgesehen der dazu eingerichtet ist, das erste Bewegungselement 17 gegenüber dem ersten Basiselement 11 zu bewegen. In der vorliegenden Ausführungsform ist dies durch eine am ersten Basiselement 11 vorgesehene Zahnstange und ein, durch den ersten Antrieb 23 angetriebenes und am ersten Bewegungselement 17 vorgesehenes Zahnrad zum Eingriff in die Zahnstange ausgestaltet. Der erste Antrieb 23 ist in bevorzugter Weise durch die Steuerungseirichtung 36 steuer- und/oder regelbar. Gegebenenfalls können zwei parallel verlaufende schienenförmige Basiselemente 11 und mehrere Führungselemente vorgesehen sein, um die Stabilität zu verbessern.

Durch die erste Antriebsvorrichtung 5 ist somit ein erster antreibbarer Freiheitsgrad zur Bewegung des Funktionskopfes 3 gegenüber der Basis 4 vorgesehen. Am ersten Bewegungselement 17 ist eine zweite Antriebsvorrichtung 6 vorgesehen. Diese umfasst ein zweites Basiselement 12, das im Wesentlichen starr mit dem ersten Bewegungselement 17 verbunden ist. Ferner umfasst die zweite Antriebsvorrichtung 6 einen zweiten Antrieb 24 zur Bewegung des zweiten Bewegungselements 18 gegenüber dem zweiten Basiselement 12. Durch die zweite Antriebsvorrichtung 6 ist somit ein weiterer antreibbarer Freiheitsgrad zur Bewegung des Funktionskopfs 3 gegenüber der Basis 4 vorgesehen. In der vorliegenden Ausführung ist die zweite Antriebsvorrichtung 6 als Linearachse ausgeführt. Diese ermöglicht eine, durch die Steuerungseinrichtung 36 gesteuerte und/oder geregelte Bewegung entlang einem linearen Freiheitsgrad. Über den Antrieb 24 wird somit das zweite Bewegungselement 18 linear gegenüber dem zweiten Basiselement 12 bewegt. Durch die vorliegende Anordnung der beiden Linearachsen der ersten und der zweiten Antriebsvorrichtung 5 und 6 ist der Funktionskopf 3 somit entlang einer Ebene zweidimensional bewegbar. In bevorzugter Weise verlaufen die Bewegungsrichtung der ersten Antriebsvorrichtung 5 und die Bewegungsrichtung der zweiten Antriebsvorrichtung 6 zueinander im Wesentlichen orthogonal. Beide Bewegungsrichtungen verlaufen in dieser Ausführungsform bevorzugt in einer Normalebene der Hauptachse.

Am zweiten Bewegungselement 18 der zweiten Antriebsvorrichtung 6 ist die dritte Antriebsvorrichtung 7 vorgesehen. Die Verbindung der dritten Antriebsvorrichtung 7 mit dem zweiten Bewegungselement 18 geschieht in der vorliegenden Ausführungsform über eine Schwenkvorrichtung 39 und eine Zustellvorrichtung 40. Die Schwenkvorrichtung 39 und die Zustellvorrichtung 40 sind im Wesentlichen starre Verbindungen, die durch Betätigung beispielsweise eines Bolzens zur groben Einstellung der Lage der dritten Antriebsvorrichtung 7 gegenüber der zweiten Antriebsvorrichtung 6 dienen. Über die Schwenkvorrichtung 39 kann der Funktionskopf 3 und die dritte Antriebsvorrichtung 7 weggeschwenkt werden um beispielsweise das Ein- oder Aussteigen der Person zu vereinfachen. Die Zustellvorrichtung 40 dient der groben Abstandseinstellung des Funktionskopfs gegenüber dem Körper 2 der Person. In bevorzugter Weise sind die Schwenkvorrichtung 39 und die Zustellvorrichtung 40 im normalen Betrieb starr und nicht angetrieben. Alternativ kann jedoch die dritte Antriebsvorrichtung, insbesondere das dritte Basiselement auch starr mit dem zweiten Bewegungselement verbunden sein. Es wird in dieser alternativen Ausführungsform auf die Schwenkvorrichtung und/oder auf die Zustellvorrichtung verzichtet.

Die dritte Antriebsvorrichtung 7 ist in der vorliegenden Ausführungsform als kraftbegrenzte Antriebsvorrichtung 29 ausgeführt. Sie weist ein drittes Basiselement 13 sowie ein drittes Bewegungselement 19 und einen dritten Antrieb 25 auf. Der dritte Antrieb 25 zur Bewegung des dritten Bewegungselements 19 gegenüber dem dritten Basiselement 13 ist als kraftbegrenzter Antrieb ausgeführt. Dazu ist grundsätzlich jeder kraftbegrenzte Antrieb geeignet, der eine Kraftbegrenzung in Systemen erhöhter oder wechselnder Beschleunigung zulässt.

Die Bewegungsrichtung der dritten Antriebsvorrichtung 7 bzw. der kraftbegrenzten Antriebsvorrichtung 29 verläuft in der dargestellten Ausführungsform orthogonal zu der Bewegungsrichtung der ersten Antriebsvorrichtung 5 und orthogonal zu der Bewegungsrichtung der zweiten Antriebsvorrichtung 6. Durch die drei angetriebenen Freiheitsgrade der ersten, zweiten und dritten Antriebsvorrichtung 5, 6, 7 ist somit eine räumliche, bevorzugt kartesische Bewegung des Funktionskopfs 3 gegenüber der Basis 4 ermöglicht. In bevorzugter Weise verläuft die Bewegungsrichtung der kraftbegrenzten Antriebsvorrichtung normal zur Richtung des Vektors der Radialbeschleunigung und somit in einer Tangentialebene der Hauptachse. Dadurch hat die Größe, der durch die Rotation des Funktionskopfs 3 und der Manipulationsanordnung 1 um die Hauptachse 32 der Bewegungsvorrichtung 30 keinen oder nur geringen Einfluss auf die Massenkräfte in der kraftbegrenzten Antriebsvorrichtung 29. In einer bevorzugten Ausführungsform verläuft die Bewegungsrichtung der kraftbegrenzten Antriebsvorrichtung 29 parallel zur Hauptachse 32 der Bewegungsvorrichtung 30.

Es entspricht jedoch durchaus dem Erfindungsgedanken die Bewegungsrichtung der kraftbegrenzten Antriebsvorrichtung 29 frei gegenüber den Beschleunigungskräften oder frei gegenüber der Hauptachse 32 zu wählen.

An der dritten Antriebsvorrichtung 7, insbesondere am dritten Bewegungselement 19 ist die vierte Antriebsvorrichtung 8 vorgesehen. Diese umfasst wiederum ein viertes Basiselement 14 und ein viertes Bewegungselement 20, wobei über einen vierten Antrieb 26 das vierte Bewegungselement 20 gegenüber dem vierten Basiselement 14 angetrieben und gedreht werden kann.

Die vierte Antriebsvorrichtung 8 ermöglicht somit einen ersten angetriebenen Drehfreiheitsgrad. An der vierten Antriebsvorrichtung 8, insbesondere am vierten Bewegungselement 20 ist eine fünfte Antriebsvorrichtung 9 vorgesehen. Diese weist einen durch einen fünften Antrieb 27 angetriebenen Drehfreiheitsgrad des fünften Bewegungselements 21 gegenüber dem fünften Basiselement 15 auf. An der fünften Antriebsvorrichtung 9 ist eine sechste Antriebsvorrichtung 10 vorgesehen, die einen sechsten Antrieb 28 umfasst, der eine Drehung des sechsten Bewegungselements 22 gegenüber dem sechsten Basiselement 16 ermöglicht. Durch die Aneinanderreihung der vierten, fünften und sechsten Antriebsvorrichtungen 8, 9, 10 ist eine kardanische Drehbarkeit des Funktionskopfs 3 ermöglicht. Dazu ist das vierte Basiselement 14 starr mit dem dritten Bewegungselement, das fünfte Basiselement 15 starr mit dem vierten Bewegungselement 20, das sechste Basiselement 16 starr mit dem fünften Bewegungselement 21 und das sechste Bewegungselement 22 starr mit dem Funktionskopf 3 verbunden.

In der vorliegenden Ausführung sind somit sechs antreibbare, steuer- und/oder regelbare Antriebsvorrichtungen seriell aneinandergereiht. Drei der Antriebsvorrichtungen erlauben eine Bewegung entlang linearer Achsen und drei der Antriebsvorrichtungen erlauben eine Drehung um Drehachsen. In bevorzugter Weise stehen jeweils zwei aufeinanderfolgende Drehachsen oder Linearachsen zueinander orthogonal. Die kraftbegrenzte Antriebsvorrichtung ist bevorzugt kraftgesteuert, die übrigen Antriebsvorrichtungen sind bevorzugt weg- oder positionsgesteuert. Die Kontaktkraft der kraftbegrenzten Antriebsvorrichtung kann dazu gewählt und/oder begrenzt werden.

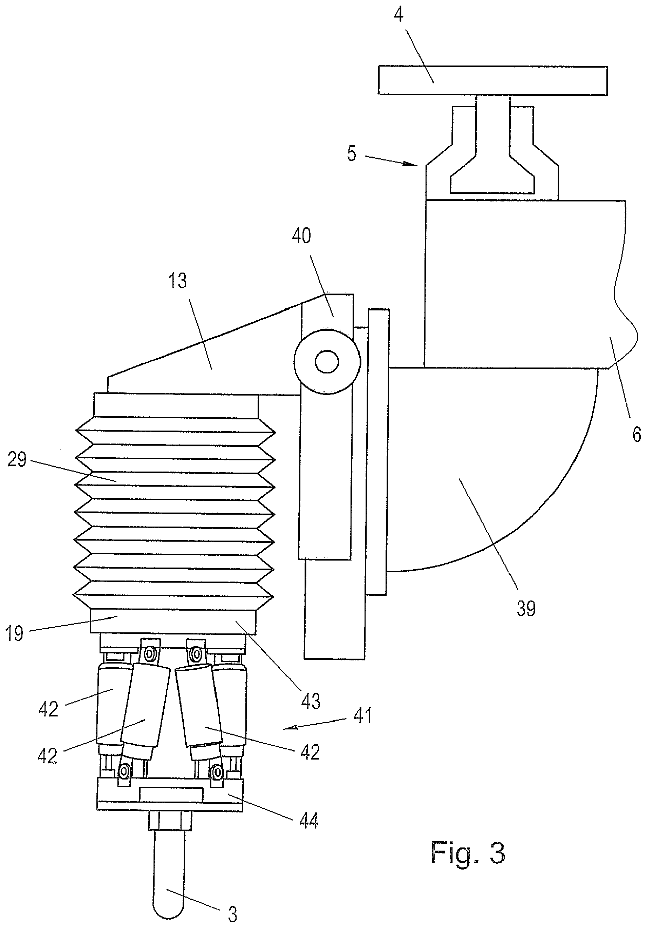

Fig. 3 zeigt eine weitere Ausführungsform eines Teils der erfindungsgemäßen Manipulatoranordnung. Bei dieser ist der Funktionskopf 3 über mehrere Antriebsvorrichtungen entlang mehrerer Freiheitsgrade bewegbar angeordnet. Der Funktionskopf ist starr oder drehbar mit dem Bewegungselement 44 einer Parallelkinematikvorrichtung verbunden oder gekoppelt. Die Parallelkinematikvorrichtung 41 umfasst ein Basiselement 43, mehrere Antriebe 42 sowie ein Bewegungselement 44, welches über die Antriebe 42 gegenüber dem Parallelkinematikvorrichtung-Basiselement 43 bewegt werden kann. Die Antriebe 42 sind in bevorzugter Weise als Linearachsen, beispielsweise als Pneumatik- oder Hydraulikzylinder ausgestaltet. Sie sind mit einer Steuerungseinrichtung verbunden und können in bevorzugter Weise getrennt voneinander oder gemeinsam gesteuert werden. Durch die unterschiedliche Längenänderung einzelner Antriebe kann eine Neigung des Bewegungselements 44 gegenüber dem Basiselement 43 erzielt werden. Auch parallele Bewegungen des Bewegungselements 44 zum Basiselement 43 ist durch geeignete Steuerung der Antriebe 42 möglich. Durch die geeignete Steuerung der Antriebe 42 ist somit eine räumliche Bewegung des Funktionskopfes 3 ermöglicht. Die Funktionsweise der Parallelkinematikvorrichtung entspricht beispielsweise der eines Hexapods oder der eines Tripods.

Das Basiselement 43 ist in der vorliegenden Ausführungsform an der kraftbegrenzten Antriebsvorrichtung 29 vorgesehen. Diese ist, wie in Fig. 2, als linear wirkende kraftbegrenzte Antriebsvorrichtung ausgeführt. Sie weist ein drittes Basiselement 13 und ein drittes Bewegungselement 19 auf, wobei das dritte Bewegungselement 19 starr mit dem Basiselement 43 verbunden ist. Das dritte Basiselement 13 ist wie in Fig. 2 mit einer Zustellvorrichtung 40 und einer Schwenkvorrichtung 39 verbunden. Die Wirkungsweise bei der Schwenkvorrichtung 39 und der Zustellvorrichtung 40 entspricht im Wesentlichen der Wirkung der äquivalenten Teile in Fig. 2. Auch der restliche Aufbau der Ausführungsform entspricht im Wesentlichen dem Aufbau aus Fig. 2. So ist die Schwenkvorrichtung starr an einer zweiten Antriebsvorrichtung vorgesehen, die ein zweites Basiselement 12, ein zweites Bewegungselement 18 und einen zweiten Antrieb 24 zur Bewegung des zweiten Bewegungselements gegenüber dem zweiten Basiselement umfasst. Das zweite Basiselement 12 ist mit einer ersten Antriebsvorrichtung 5 verbunden. Auch diese entspricht von ihrem Aufbau der ersten Antriebsvorrichtung aus Fig. 2. Die erste Antriebsvorrichtung 5 ist an der Basis 4 vorgesehen. Diese Basis 4 kann, wie in Fig. 1 dargestellt, verschiebbar oder fest mit der Bewegungsvorrichtung 30 verbunden sein.

Die vorliegende Ausführungsform der Fig. 3 unterscheidet sich von der Ausführungsform der Fig. 2 im Wesentlichen durch die Ausgestaltung der Antriebsvorrichtungen zur Bewegung des Funktionskopfs gegenüber der kraftbegrenzten Antriebsvorrichtung 29. Während in Fig. 2 eine Rotation mehrerer Elemente um im Wesentlichen orthogonal zueinander stehende Achsen ermöglicht ist, ist gemäß der Ausführungsform der Fig. 3 eine Parallelkinematikvorrichtung für die Neigung und die räumliche Bewegung des Funktionskopfs 3 eingesetzt.

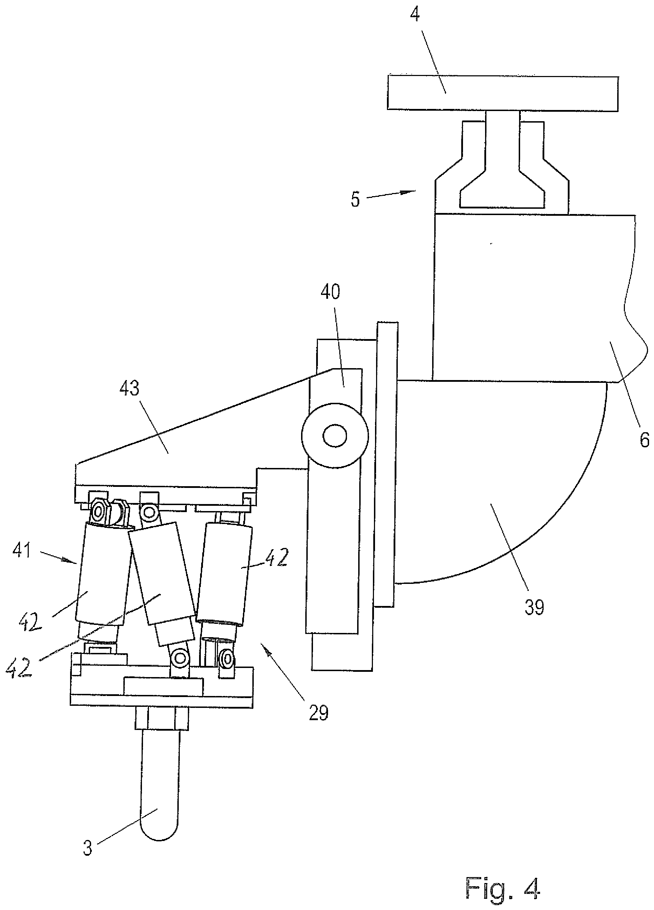

Fig. 4 zeigt eine weitere Ausführungsform der erfindungsgemäßen Vorrichtung. Dabei sind wiederum an einer Basis 4 eine erste Antriebsvorrichtung 5 sowie eine zweite Antriebsvorrichtung 6 vorgesehen. An der zweiten Antriebsvorrichtung 6 sind wiederum eine Schwenkvorrichtung 39 sowie eine Zustellvorrichtung 40 vorgesehen. Die Zustellvorrichtung 40 ist starr mit dem Basiselement 43 verbunden. Der Aufbau der Manipulatoranordnung entspricht bezüglich der ersten Antriebsvorrichtung 5, der zweiten Antriebsvorrichtung 6 sowie der Schwenkvorrichtung 39 und der Zustellvorrichtung 40 dem Aufbau der Ausführungsformen der Figuren 2 und 3. Gemäß der Ausführungsform nach Fig. 4 ist die kraftbegrenzte Antriebsvorrichtung 29 als Parallelkinematikvorrichtung 41 ausgeführt. Dabei kann jeder einzelne Antrieb 42 der Parallelkinematikvorrichtung 41 kraftbegrenzt oder sensitiv ausgeführt sein. Alternativ dazu kann auch die ganze Parallelkinematikvorrichtung 41 als eine Einheit kraftbegrenzt ausgeführt sein. Die Ausführungsform der Fig. 4 entspricht somit im Wesentlichen der Ausführungsform der Fig. 3, wobei die Anordnung der kraftbegrenzten Antriebsvorrichtung mit einer Parallelkinematikvorrichtung in einer kraftbegrenzten Parallelkinematikvorrichtung vereint ist.

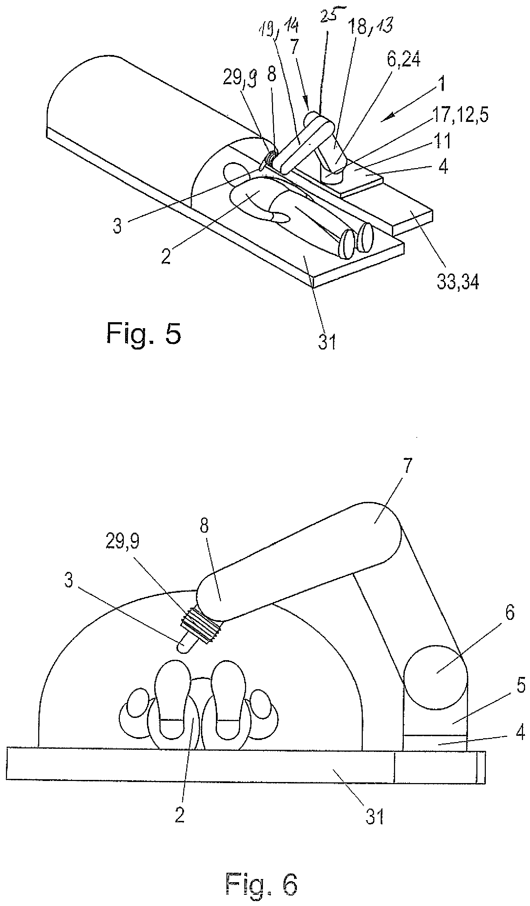

Fig. 5 zeigt eine mögliche weitere Ausführungsform der erfindungsgemäßen Manipulatoranordnung in einer schematisierten Darstellung. Die Befestigungsvorrichtung 31 zur Aufnahme oder zur Befestigung eines Körpers 2 ist dabei ähnlich der Befestigungsvorrichtung aus Fig. 1 angeordnet. Beispielsweise verläuft die Körperhochachse radial, wobei der Kopf Richtung Hauptachse 32 weist. Die Beschleunigungskomponente, die durch die Drehung der Befestigungsvorrichtung 31 um die Hauptachse 32 erzeugt wird, verläuft ebenfalls im Wesentlichen der Körperhochachse folgend. Die Manipulatoranordnung 1 umfasst in der vorliegenden Ausführung eine Basis 4, die an dem ersten oder zweiten Trägerelement 33, 34 der Bewegungsvorrichtung verschiebbar oder fest angeordnet ist. Ferner umfasst die Manipulatoranordnung eine erste Antriebsvorrichtung 5 mit einem ersten Basiselement 11 das fest mit der Basis 4 verbunden ist. Über einen ersten Antrieb 23 ist das erste Bewegungselement 17 gegenüber dem ersten Basiselement 11 bewegbar und antreibbar. Die Bewegung erfolgt durch Rotation um eine Achse, die im Wesentlichen parallel zur Hauptachse 32 verläuft. An dem ersten Bewegungselement 17 ist ein zweites Basiselement 12 vorgesehen. Gegenüber diesem zweiten Basiselement 12 kann ein zweites Bewegungselement 18 über einen zweiten Antrieb 24 bewegt werden. Zusammen ergeben die Komponenten die zweite Antriebsvorrichtung 6. Auch dieser Antrieb ist ein Rotationsantrieb, wobei die Achse der Rotation im Wesentlichen normal zur Achse der ersten Antriebsvorrichtung 5 verläuft. Das zweite Bewegungselement 18 ist armförmig ausgeführt und ist in einem Bereich entfernt vom zweiten Antrieb mit der dritten Antriebsvorrichtung 7 verbunden. Diese umfasst wiederum ein drittes Basiselement 13, das mit dem zweiten Bewegungselement 18 starr verbunden ist. Ferner umfasst die dritte Antriebsvorrichtung einen dritten Antrieb 25, der ein armförmiges drittes Bewegungselement 19 um eine Drehachse drehen kann, die im Wesentlichen normal zu der Drehachse der zweiten Antriebsvorrichtung 6 verläuft. Das dritte Basiselement 19 und das zweite Bewegungselement können in einer bevorzugten Ausführung einstückig ausgeführt sein.

An der dritten Antriebsvorrichtung 7, insbesondere am dritten Bewegungselement 19 ist eine vierte Antriebsvorrichtung 8 vorgesehen. An der vierten Antriebsvorrichtung 8 ist eine fünfte Antriebsvorrichtung 9 vorgesehen die als kraftbegrenzte Antriebsvorrichtung 29 ausgeführt ist.

Auch Fig. 6 zeigt dieselbe Anordnung wie Fig. 5 mit einer Basis 4, an der drehbar die erste Antriebsvorrichtung 5 vorgesehen ist. An der ersten Antriebsvorrichtung 5 ist die zweite Antriebsvorrichtung 6 vorgesehen, an der zweiten Antriebsvorrichtung 6 ist die dritte Antriebsvorrichtung 7 vorgesehen, an der dritten Antriebsvorrichtung 7 ist die vierte Antriebsvorrichtung 8 vorgesehen und an der vierten Antriebsvorrichtung 8 ist die fünfte Antriebsvorrichtung 9 vorgesehen, die als kraftbegrenzte Antriebsvorrichtung 29 ausgeführt ist. In den Fig. 5 und 6 ist an der kraftbegrenzten Antriebsvorrichtung 29 der Funktionskopf 3 vorgesehen. Über die Bewegung der Manipulatoranordnung 1, insbesondere die Bewegung der Bewegungselemente 17, 18, 19, 20 durch die Antriebe 23, 24, 25, 26, 27 und 29 kann der Funktionskopf an den Körper 2 der Person geführt werden.

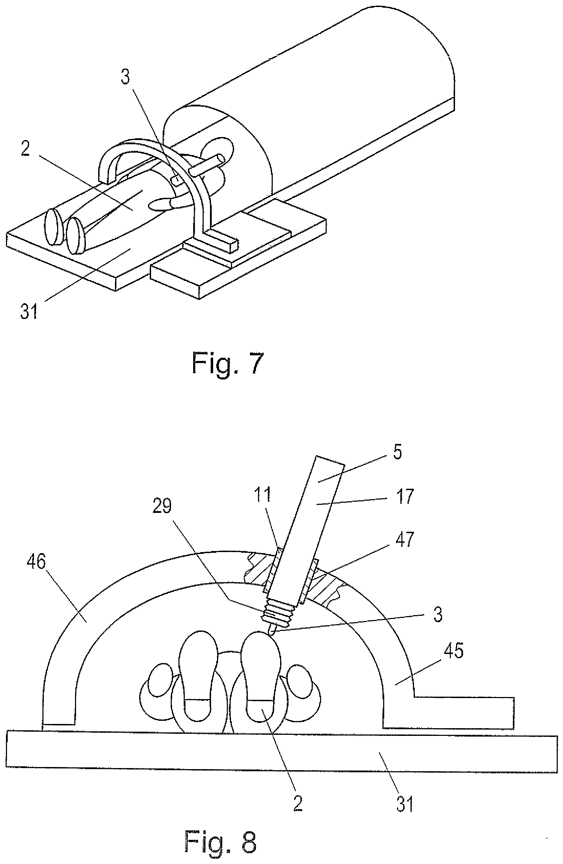

Fig. 7 und Fig. 8 zeigen jeweils unterschiedliche Ansichten einer weiteren Ausführungsform der erfindungsgemäßen Anordnung einer Manipulatoranordnung und einer Bewegungsvorrichtung. Wiederum entspricht die Lage der Befestigungsvorrichtung 31 für den Körper 2 der Lage aus Fig. 1. Die Manipulatoranordnung zur Bewegung eines Funktionskopfs 3 an den Körper 2 der Person umfasst einen Bogenantrieb 45. Der Bogenantrieb 45 weist ein Basiselement 46 auf, das sich bogenförmig um oder über den Körper 2 der Person erstreckt. Verschiebbar an diesem angeordnet ist das Bewegungselement 47 des Bogenantriebs, wobei der Funktionskopf 3 im Wesentlichen nach innen Richtung Körper 2 zeigt.

An dem Bewegungselement 47 des Bogenantriebs 45 ist eine erste Antriebsvorrichtung 5 vorgesehen. Diese umfasst ein erstes Basiselement 11 sowie ein erstes Bewegungselement 17. Dieses ist linear gegenüber dem ersten Basiselement bewegbar. Die Richtung der Linearbewegung folgt im Wesentlichen der radialen Richtung der momentanen Position entlang des Basiselements 46 des Bogenantriebs.

In Verlängerung des ersten Bewegungselements 17 ist die kraftbegrenzte Antriebsvorrichtung 29 vorgesehen. An dieser ist der Funktionskopf 3 vorgesehen, der in Richtung des Körpers 2 weist und durch die Manipulatoranordnung an diesen zuführbar und andrückbar ist.

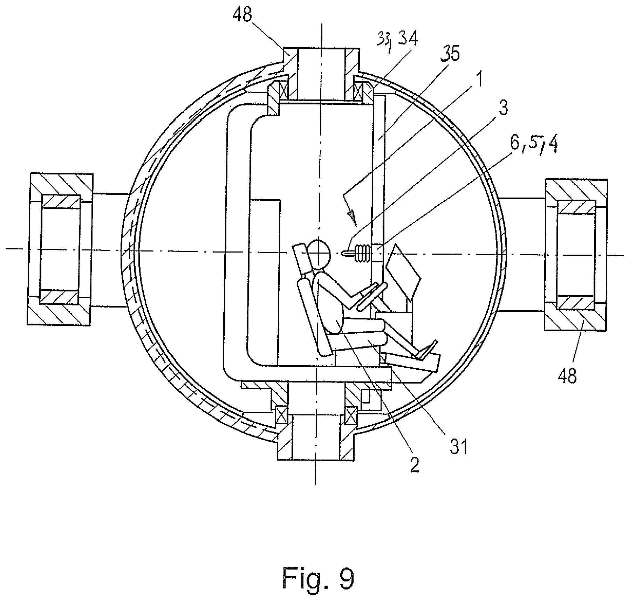

Fig. 9 zeigt eine weitere Anwendung einer erfindungsgemäßen Manipulatoranordnung und einer erfindungsgemäßen Bewegungsvorrichtung.

Die Befestigungsvorrichtung 31 für den Körper 2 einer Person ist dabei in oder an einer Simulatorkabine vorgesehen. Die Simulatorkabine ist gemäß dem Stand der Technik beispielsweise an einer Ein- oder Mehrarmzentrifuge vorgesehen. Dazu ist die Simulatorkabine über eine oder mehrere Rollringe 48 drehbar und antreibbar gelagert. Der äußerste Rollring ist bevorzugt mit dem Arm einer Zentrifuge verbunden. Dieser Arm wird gemäß dem Stand der Technik um eine Hauptachse 32 gedreht. Die Simulatorkabine ist dabei in einem gewissen Normalabstand zur Drehachse 32 angeordnet. In der Simulatorkabine, bevorzugt im Bereich der Befestigungsvorrichtung 31 ist wiederum eine Manipulatoranordnung 1 vorgesehen. Diese ist in der vorliegenden Ausführung schematisch dargestellt. Die Manipulatoranordnung umfasst wiederum einen Funktionskopf 3, der über mehrere antreibbare Antriebsvorrichtungen an den Körper 2 der Person führbar ist. Zumindest eine Antriebsvorrichtung ist als kraftbegrenzte Antriebsvorrichtung 29 ausgeführt.

Über eine erste Antriebsvorrichtung 5 ist der Funktionskopf 3 linear verfahrbar. Über eine zweite Antriebsvorrichtung 6 kann der Funktionskopf in einer weiteren Richtung linear verfahren werden. In bevorzugter Weise ist an der Basis eine Manipulatoranordnung gemäß der vorliegenden Ausführung der Fig. 9 oder gemäß einer Ausführungen der Fig. 1 bis 8 vorgesehen.

Gemäß einer weiteren Ausführungsform sind mehrere Antriebsvorrichtungen als kraftbegrenzte Antriebsvorrichtungen ausgeführt. Diese Ausführungsform entspricht der Ausführungsform der Fig.2, wobei zusätzlich zur dritten Antriebsvorrichtung auch die zweite Antriebsvorrichtung als kraftbegrenzte Antriebsvorrichtung ausgeführt ist.

Gemäß einer weiteren Ausführungsform sind zusätzlich zur dritten Antriebsvorrichtung der Fig.2 auch die zweite und die erste Antriebsvorrichtung als kraftbegrenzte Antriebsvorrichtungen ausgeführt ist.

Gemäß einer weiteren Ausführungsform sind jene Antriebsvorrichtungen als kraftbegrenzte Antriebsvorrichtungen ausgeführt, deren Bewegungsachsen jeweils im Wesentlichen in einer Tangentialebene der Hauptachse liegen. In Fig. 2 liegen z.B. die Bewegungsachsen der ersten und der dritten Antriebsvorrichtung jeweils in einer Tangentialebene der Hauptachse.

In weiterer Folge wird eine exemplarische Verwendung der erfindungsgemäßen Manipulatoranordnung und der erfindungsgemäßen Bewegungsvorrichtungen zur Ultraschalluntersuchung bei erhöhter Beschleunigung weiter erörtert. Zur Untersuchung oder Behandlung eines Körpers 2 ist der Körper an der Befestigungsvorrichtung 31 befestigt oder auf dieser platziert. Die Befestigungsvorrichtung 31 ist mit einem ersten Trägerelement 33 oder mit einem zweiten Trägerelement 34 verbunden. Bei ruhender Bewegungsvorrichtung legt oder setzt sich die Person auf die Befestigungsvorrichtung 31 und schnallt sich gegebenenfalls an dieser fest. Im Falle von Einarmzentrifugen, Flugsimulatoren und/oder Simulatorkabinen, wie beispielsweise in Fig. 9 dargestellt, setzt sich die Person in ein nachgebildetes Cockpit eines Flugzeuges. In der Bewegungsvorrichtung nach Fig. 1 legt sich die Person auf die Befestigungsvorrichtung, die gegebenenfalls Ergometer-Elemente oder andere Vorrichtungen zur Belastung des menschlichen Körpers aufweist.

In weiterer Folge wird die Manipulatoranordnung in den Bereich des Körpers 2 der Person gebracht. Dies geschieht beispielsweise über die Verschiebung der Basis 4 entlang des Basisträgerelements 35 oder durch die ferngesteuerte oder automatisierte Bedienung der Antriebsvorrichtungen, beispielsweise der ersten Antriebsvorrichtung 5. Der Funktionskopf 3 wird dabei an die gewünschte Stelle des Körpers geführt und angedrückt.
Zur Ultraschalluntersuchung ist der Funktionskopf als Ultraschallkopf ausgeführt. Dieser wird in ruhender Stellung der Bewegungsvorrichtung derart an den Körper angelegt, dass das gewünschte Bild an einer Auswerte-Einheit, beispielsweise in einem Kontrollraum oder an einem mobilen Gerät, angezeigt wird. In dieser Grundstellung, bei der der Funktionskopf über die Manipulatoranordnung an die gewünschte Stelle des Körpers 2 geführt ist wird nun die Bewegung der Bewegungsvorrichtung gestartet. Im Falle der Ausführungsform der Fig.1 drehen sich das erste und das zweite Trägerelement 33, 34 um die Hauptachse 32. In anderen Ausführungsformen, beispielsweise in der Ausführungsform der Fig.9 wird die dargestellte Simulatorkabine über einen Hauptarm - nicht dargestellt - ebenfalls um eine Hauptachse 32 gedreht. Durch die erhöhte und/oder wechselnde Beschleunigung ändern gegebenenfalls auch die betrachteten Organe des Körpers 2 oder der Körpers selbst die Lage. Um nun das gewünschte Bild zu bekommen, kann über die Manipulatoranordnung der Funktionskopf bewegt werden. Dies geschieht über die ferngesteuerte oder automatisierte Bewegung der Antriebsvorrichtungen. Zur Steuerung der Bewegung des Funktionskopfes und der Manipulatoranordnung sind eine Steuerungseinrichtung 36 und eine Dateneingabeanordnung 37 vorgesehen. Beispielsweise kann eine weitere Person aus einem Kontrollraum über Elemente wie Joysticks, Schieberegler, Datenhandschuhe, etc. die Bewegung des Funktionskopfs gegenüber dem Körper 2 steuern. Dazu ist der Funktionskopf gemäß der Manipulatoranordnungen der Figuren 2 bis 8 entlang mehrerer Freiheitsgrade antreibbar und bewegbar. So kann einerseits über rotatorische Freiheitsgrade der Winkel des Funktionskopfs gegenüber dem Körper verändert werden. Andererseits kann über translatorische Freiheitsgrade die Position des Funktionskopfes gegenüber dem Körper verändert werden. Um nun eine andere Stelle des Körpers zu untersuchen kann über die Dateneingabeanordnung und die Steuerung der Antriebsvorrichtungen der Funktionskopf von dem Körper 2 abgehoben und an eine andere Stelle des Körpers geführt und angedrückt werden.

Durch die kraftbegrenzte Antriebsvorrichtung ist dabei eine unabsichtliche Verletzung des Körpers verhindert. In bevorzugter Weise ist jene Antriebsvorrichtung kraftbegrenzt ausgeführt, deren Freiheitsgrad oder Wirkrichtung eine Druckausübung auf den Körper 2 ermöglicht. Beispielsweise ist das jener lineare Freiheitsgrad, dessen Wirkrichtung normal zur Körperoberfläche verläuft. Wird nun der Funktionskopf über die Fernsteuerung an den Körper 2 geführt, so geschieht dies mit einer voreingestellten oder gewählten Kraft und einer voreingestellten oder gewählten Maximalkraft. Selbst bei einer Fehlfunktion jener Antriebsvorrichtungen, die nicht kraftbegrenzt sind, ist eine ungewollte Verletzung des Körpers durch den Funktionskopf verhindert, da die maßgebliche Kraftkomponente der kinematischen Anordnung kraftbegrenzt ausgeführt ist.

Zur Durchführung anderer Untersuchungen oder Behandlungen kann der Funktionskopf Komponenten, Untersuchungs- und/oder Behandlungsvorrichtungen wie beispielsweise einen Ultraschallmesskopf, optische Aufnahmegeräte, akustische Aufnahmegeräte, Widerstandsmessgeräte, eine Injektionsanordnung, eine Flüssigkeitsanalyseanordnung, eine Blutabnahmevorrichtung, eine Analysevorrichtung, eine chemische Analysevorrichtung, eine Strahlenquelle z.B. Röntgen-, Gamma- oder Infrarotstrahlung, eine Laserquelle, Probenentnahmevorrichtungen, Temperaturmessgeräte, Strommessgeräte, Strahlendetektionsvorrichtungen und/oder weitere radiologische, invasive oder berührende Vorrichtungen zu diagnostischen oder therapeutischen Zwecken umfassen. Die Bewegung des Funktionskopfs und die Durchführung der Untersuchung oder der Bahndlung geschieht in beschriebener Weise.

Die Bewegungsvorrichtungen die in Fig. 1 und Fig. 9 dargestellt sind, sind exemplarische Bewegungsvorrichtungen. An diesen ist die erfindungsgemäße Manipulatoranordnung vorgesehen. Die erfindungsgemäße Manipulatoranordnung weist mehrere Antriebsvorrichtungen auf, wobei zumindest eine dieser Antriebsvorrichtungen als kraftbegrenzte Antriebsvorrichtung ausgeführt ist. Die Manipulatoranordnung umfasst einen Funktionskopf der eingerichtet ist, um berührende und/oder invasive Untersuchung oder Behandlungen am tierischen oder menschlichen Körper vorzunehmen. Dazu ist der Funktionskopf in bevorzugter Weise entlang mehrerer translatorischer Freiheitsgrade verfahrbar und um mehrere rotatorische Freiheitsgrade drehbar. Die unterschiedlichen Ausführungen der Freiheitsgrade der Figuren 2, 3 und 4, insbesondere die Ausführung der kraftbegrenzten Antriebsvorrichtung mit einer kardanischen Antriebsvorrichtung, einer Parallelkinematikanordnung oder einer kraftbegrenzten Parallelkinematikanordnung sind auch an den in den Figuren 5 bis 8 dargestellten Manipulatoranordnungen anordbar. So kann beispielsweise die kraftbegrenzte Parallelkinematikanordnung der Fig. 4 an dem Roboterarm der Fig. 6 angeordnet werden. Beispielsweise kann auch die Parallelkinematikanordnung mit daran angeordneter eigenständiger, kraftbegrenzter Antriebsvorrichtung an dem Bewegungselement des Bogenantriebs 47 der Figuren 7 und 8 vorgesehen sein. Kombinationen der Figuren 2, 3, 4 mit den unterschiedlichen Ausführungen der Manipulatoranordnungen in den Figuren 2 und 5 bis 8 entsprechen ebenfalls dem Erfindungsgedanken. Die Manipulatoranordnungen gemäß der Figuren 2 bis 8 und/oder dem allgemeinen Beschreibungsteil sind an den Bewegungsvorrichtungen beispielsweise der Figuren 1 und 9 oder des allgemeinen Beschreibungsteils anbringbar.

### Bezugszeichenliste:

- 1: Manipulatoranordnung
- 2: Körper
- 3: Funktionskopf
- 4: Basis
- 5: Erste Antriebsvorrichtung (Y)
- 6: Zweite Antriebsvorrichtung (X)
- 7: Dritte Antriebsvorrichtung (Z)
- 8: Vierte Antriebsvorrichtung
- 9: Fünfte Antriebsvorrichtung
- 10: Sechste Antriebsvorrichtung
- 11: Erstes Basiselement
- 12: Zweites Basiselement
- 13: Drittes Basiselement
- 14: Viertes Basiselement
- 15: Fünftes Basiselement
- 16: Sechstes Basiselement
- 17: Erstes Bewegungselement
- 18: Zweites Bewegungselement
- 19: Drittes Bewegungselement
- 20: Viertes Bewegungselement
- 21: Fünftes Bewegungselement
- 22: Sechstes Bewegungselement Erster Antrieb
- 23: Erster Antrieb
- 24: Zweiter Antrieb
- 25: Dritter Antrieb
- 26: Vierter Antrieb
- 27: Fünfter Antrieb
- 28: Sechster Antrieb
- 29: Kraftbegrenzte Antriebsvorrichtung
- 30: Bewegungsvorrichtung
- 31: Befestigungsvorrichtung
- 32: Hauptachse
- 33: Erstes Trägerelement
- 34: Zweites Trägerelement
- 35: Basisträgerelement
- 36: Steuerungseinrichtung
- 37: Dateneingabeanordnung
- 38: Führungselement
- 39: Schwenkvorrichtung
- 40: Zustellvorrichtung
- 41: Parallelkinematikvorrichtung
- 42: Antrieb - Parallelkinematikvorrichtung
- 43: Basiselement Parallelkinematikvorrichtung
- 44: Bewegungselement Parallelkinematikvorrichtung
- 45: Bogenantrieb
- 46: Basiselement Bogenantrieb
- 47: Bewegungselement Bogenantrieb
- 48: Rollring

## Patentansprüche

1. Bewegungsvorrichtung mit zumindest einer Befestigungsvorrichtung (31) zur Aufnahme, Anlage und/oder Befestigung eines menschlichen oder tierischen Körpers (2), die an einem ersten Trägerelement (33) vorgesehen ist, das um eine erste Hauptachse (32) drehbar angeordnet ist,
wobei die Bewegungsvorrichtung als Zentrifuge, medizinische Zentrifuge oder Trainingszentrifuge ausgebildet ist,
**dadurch gekennzeichnet,**
∘ dass eine Manipulatoranordnung (1) an der Bewegungsvorrichtung angeordnet ist,
∘ dass die Manipulatoranordnung (1) zur berührenden und/oder invasiven Untersuchung oder Behandlung am menschlichen oder tierischen Körper (2) unter dem Einfluss dauerhaft erhöhter und/oder wechselnder Beschleunigung geeignet ist,
wobei eine erhöhte und/oder wechselnde Beschleunigung ein Beschleunigungszustand ist, bei dem die Manipulatoranordnung (1) erhöhte und/oder wechselnde Beschleunigungskräfte erfährt und bewegt wird,
∘ dass die Manipulatoranordnung (1) einen Funktionskopf (3) umfasst, wobei durch die Bewegung und durch die Änderung der Bewegung auf die Manipulatoranordnung (1) und auf den Funktionskopf Beschleunigungskräfte wirken, die von den Beschleunigungskräften der Umgebung abweichen,
∘ dass der Funktionskopf (3) relativ zu einer Basis (4) entlang mehrerer durch Antriebsvorrichtungen bewegbarer Freiheitsgrade bewegbar ist, wobei die Lage und/oder die Kontaktkraft des den Körper (2) berührenden Funktionskopfs (3) gegenüber dem Körper (2) unter dem Einfluss erhöhter und/oder wechselnder Beschleunigung veränderbar ist,
∘ dass zumindest eine Antriebsvorrichtung als kraftbegrenzte Antriebsvorrichtung (29) ausgeführt ist und dass die Kontaktkraft durch die kraftbegrenzte Antriebsvorrichtung systeminhärent begrenzt ist, wodurch eine unabsichtliche Verletzung des Körpers durch den Funktionskopf und/oder die Manipulatoranordnung (1) ausgeschlossen ist und insbesondere, dass die Kontaktkraft über ein Druckventil der kraftbegrenzten Antriebsvorrichtung begrenzt ist.

2. Bewegungsvorrichtung nach Anspruch 1, **dadurch gekennzeichnet, dass** die kraftbegrenzte Antriebsvorrichtung (29) eine oder mehrere kolbenlose pneumatische Aktoren, eine oder mehrere Luftmuskelanordnungen, eine oder mehrere Luftbalganordnungen, eine oder mehrere Pneumatikzylinderanordnungen mit Pneumatikzylindern mit im Wesentlichen haftreibungsfrei gelagerten Kolben, eine oder mehrere getriebelose elektrische Lineareinheiten mit im Wesentlichen haftreibungsfrei gelagerten Ankern und/oder eine oder mehrere Führungen umfasst.

3. Bewegungsvorrichtung nach einem der Ansprüche 1 oder 2, **dadurch gekennzeichnet, dass** die Antriebsvorrichtungen eine Linearachse, einen Zahnstangenantrieb, einen Parallelkinematikantrieb, einen Hexapod, einen Tripod, einen Roboterarm, einen Rotationsantrieb, einen kardanischen Antrieb und/oder einen kartesischen Antrieb umfassen.

4. Bewegungsvorrichtung nach einem der Ansprüche 1 bis 3, **dadurch gekennzeichnet, dass** die Antriebsvorrichtungen jeweils ein Basiselement, ein Bewegungselement und einen Antrieb zur Bewegung des Bewegungselements gegenüber dem Basiselement entlang des oder der jeweiligen Freiheitsgrade umfassen und dass die Antriebsvorrichtungen seriell aneinandergereiht sind, wobei jeweils das Bewegungselement einer Antriebsvorrichtung mit dem Basiselement der darauffolgenden Antriebsvorrichtung verbunden oder gekoppelt ist.

5. Bewegungsvorrichtung nach einem der Ansprüche 1 bis 4, **dadurch gekennzeichnet, dass** die Antriebsvorrichtungen von einer oder mehreren Steuerungseinrichtungen(36) gesteuert und/oder geregelt sind und dass die Antriebsvorrichtungen und die Steuerungseinrichtungen(36) zum Betrieb bei erhöhter und/oder wechselnder Beschleunigung eingerichtet sind.

6. Bewegungsvorrichtung nach einem der Ansprüche 1 bis 5, **dadurch gekennzeichnet, dass** der Funktionskopf (3) in wählbarer Stellung an den Körper (2) führbar ist.

7. Bewegungsvorrichtung nach einem der Ansprüche 1 bis 6, **dadurch gekennzeichnet, dass** die Bewegung des Funktionskopfes (3) gegenüber der Basis (4) fernsteuerbar und/oder automatisierbar ist, insbesondere über eine oder mehrere Steuerungseinrichtungen(36) und/oder eine oder mehrere Dateneingabeanordnungen(37) fernsteuerbar und/oder automatisierbar ist.

8. Bewegungsvorrichtung nach einem der Ansprüche 1 bis 7, **dadurch gekennzeichnet, dass** die Dateneingabeanordnung (37) Eingabegeräte wie Joysticks, Schieberegler, Datenhandschuhe, Computerprogramme, automatisierte Programme und/oder ähnliche Anordnungen umfasst.

9. Bewegungsvorrichtung nach einem der Ansprüche 1 bis 8, **dadurch gekennzeichnet, dass** der Funktionskopf (3) Komponenten, Untersuchungs- und/oder Behandlungsvorrichtungen wie beispielsweise einen Ultraschallmesskopf, ein optisches Aufnahmegerät, ein akustisches Aufnahmegerät, ein Widerstandsmessgerät, eine Injektionsanordnung, eine Flüssigkeitsanalyseanordnung, eine Blutabnahmevorrichtung, eine Analysevorrichtung, eine chemische Analysevorrichtung, eine Strahlenquelle für beispielsweise Röntgen-, Gamma- oder Infrarotstrahlung, eine Laserquelle, eine Probenentnahmevorrichtung, ein Temperaturmessgerät, ein Strommessgerät, eine Strahlendetektionsvorrichtung, ein endoskopisches Untersuchungsgerät, eine Vorrichtung zur optischen Augenuntersuchung und/oder weitere radiologische, invasive oder berührende Vorrichtungen zu diagnostischen oder therapeutischen Zwecken umfasst.

10. Bewegungsvorrichtung nach einem der vorangegangenen Ansprüche, **dadurch gekennzeichnet, dass** zur Steuerung der Bewegung des Funktionskopfes eine Multiachssteuerung vorgesehen ist, bei der über eine oder mehrere Dateneingabeanordnungen mehrere Achsen gleichzeitig steuerbar sind, und wobei durch Transformation des Steuerungskoordinatensystems auf einen beliebigen Punkt die Bewegungscharakteristik der Manipulatoranordnung anpassbar ist, wobei der Steuerungspunkt bevorzugt in einem Berührpunkt des Funktionskopfs mit dem Körper gelegt ist.

11. Bewegungsvorrichtung nach einem der Ansprüche 1 bis 10, **dadurch gekennzeichnet, dass** die Bewegungsvorrichtung als Flugsimulator, als Einarmzentrifuge, als Zentrifuge mit verfahrbarem Schlitten, als Zentrifuge mit verfahrbarem Heaveschlitten, als Trainingszentrifuge, als Trainingszentrifuge zum Einsatz unter Schwerelosigkeit, als medizinische Zentrifuge oder als medizinische Zentrifuge mit mehreren um eine erste Drehachse (32) drehbar angeordneten Nacellen ausgeführt ist.

12. Bewegungsvorrichtung nach einem der Ansprüche 1 bis 11, **dadurch gekennzeichnet, dass** ein mit dem ersten Trägerelement (33) gekoppeltes oder verbundenes Basisträgerelement (35) vorgesehen ist, das mit der Basis(4) der Manipulatoranordnung(1) verbunden, gekoppelt oder verbindbar ist.

13. Bewegungsvorrichtung nach einem der Ansprüche 1 bis 12, **dadurch gekennzeichnet, dass** der Funktionskopf (3) über die kraftbegrenzte Antriebsvorrichtung(29) oder über die kraftbegrenzten Antriebsvorrichtungen(29) im Wesentlichen entlang einer Tangentialebene der Hauptachse (32) bewegbar ist.

14. Bewegungsvorrichtung nach einem der Ansprüche 1 bis 13, **dadurch gekennzeichnet, dass** der Funktionskopf (3) während der Drehung um die Hauptachse (32) über die Betätigung der Manipulatoranordnung (1) durch eine Dateneingabeanordnung (37) ferngesteuert oder automatisiert an den Körper (2) führbar und/oder zum Körper positionierbar ist.

15. Bewegungsvorrichtung nach einem der Ansprüche 1 bis 14, **dadurch gekennzeichnet, dass** der Funktionskopf (3) während der Drehung um die Hauptachse (32) über die Betätigung der Manipulatoranordnung (1) durch eine Dateneingabeanordnung (37) ferngesteuert oder automatisiert in wählbarer Stellung und/oder mit wählbarer Kontaktkraft an den Körper (2) führbar und andrückbar und/oder zum Körper positionierbar ist.

16. Bewegungsvorrichtung nach einem der Ansprüche 1 bis 15, **dadurch gekennzeichnet, dass** die Stellung und/oder die Kontaktkraft zwischen dem Funktionskopf (3) und dem Körper (2) während der Drehung um die Hauptachse (32) über die Betätigung der Manipulatoranordnung (1) durch eine Dateneingabeanordnung (37) ferngesteuert oder automatisiert veränderbar ist.

17. Bewegungsvorrichtung nach einem der Ansprüche 1 bis 16, **dadurch gekennzeichnet, dass** die Kontaktkraft wählbar ist und dass die Kontaktkraft über ein Druckventil der kraftbegrenzten Antriebsvorrichtung begrenzt ist, wobei das Druckventil als Regelventil ausgeführt ist.

## Claims

1. A movement device comprising at least one fastening device (31) for receiving, holding and/or fastening a human or animal body (2), which is provided on a first carrier element (33) arranged rotatably about a first main axis (32),
wherein the movement device is embodied as a centrifuge, a medical centrifuge or a training centrifuge,
**characterized in that**
∘ a manipulator configuration (1) is provided on the movement device,
∘ the manipulator configuration (1) is suitable for touching and/or invasive examination or treatment of the human or animal body (2) under the influence of elevated and/or variable acceleration,
wherein an elevated and/or variable acceleration is an acceleration condition, in which the manipulator configuration (1) is exposed to elevated and/or variable acceleration forces and is moved,
∘ the manipulator configuration (1) comprises a function head (3),
wherein acceleration forces that are different from the ambient acceleration forces act on the manipulator configuration (1) and on the function head due to the movement and due to the change in the movement,
∘ the function head (3) is movable in relation to a base (4) along a plurality of degrees of freedom that are movable by drive devices, wherein the position and/or the contact force of the function head (3) touching the body (2) can be changed with respect to the body (2) under the influence of elevated and/or variable acceleration,
∘ at least one drive device is configured as a force-limited drive device (29), and the contact force is system inherently limited by the force-limited drive device, so that accidental injury of the body by the function head and/or the manipulator configuration (1) is prevented, and in particular the contact force is limited by a pressure valve of the force-limited drive device.

2. The movement device according to claim 1, **characterized in that** the force-limited drive device (29) comprises one or more pistonless pneumatic actuators, one or more pneumatic muscle configurations, one or more air bellows configurations, one or more pneumatic cylinder configurations having pistons in an essentially frictionless mount, one or more gearless electric linear units having armatures in essentially frictionless mounts and/or one or more guides.

3. The movement device according to any one of claims 1 or 2, **characterized in that** the drive devices comprise a linear axle, a rack and pinion drive, a parallel kinematic drive, a hexapod, a tripod, a robot arm, a rotational drive, a cardan drive and/or a cartesian drive.

4. The movement device according to any one of claims 1 through 3, **characterized in that** the drive devices comprise a base element, a movement element and a drive for moving the movement element with respect to the base element along the degrees of freedom or the respective degrees of freedom, and the drive devices are arranged serially, wherein the movement element of a drive device is coupled or connected to the base element of the following drive device.

5. The movement device according to any one of claims 1 through 4, **characterized in that** the drive devices are controlled and/or closed loop controlled by one or more control devices (36), and the drive devices and the control devices (36) are configured for operation at an increased and/or variable acceleration.

6. The movement device according to any one of claims 1 through 5, **characterized in that** the function head (3) is guided to the body (2) in a selectable position.

7. The movement device according to any one of claims 1 through 6, **characterized in that** the movement of the function head (3) with respect to the base (4) is remote controlled and/or automatable, in particular being remote controlled and/or automatable by means of one or more control devices (36) and/or one or more data input configurations (37).

8. The movement device according to any one of claims 1 through 7, **characterized in that** the data input configuration (37) comprises joysticks, slide bars, data gloves, computer programs, automated programs and/or similar configurations.

9. The movement device according to any one of claims 1 through 8, **characterized in that** the function head (3) comprises components, examination and/or treatment devices such as an ultrasonic measurement head, an optical recording device, an acoustic recording device, a resistance measuring device, an injection configuration, a liquid analysis configuration, a blood sampling device, an analysis device, a chemical analysis device, a radiation source for example for X-ray, gamma ray or infrared radiation, , a laser source, a sampling device, a temperature gauge, an electric ammeter, a radiation detecting device, an endoscopic examination instrument, an instrument for optical examination of the eyes and/or additional radiologic, invasive or contact instruments for diagnostic or therapeutic purposes.

10. The movement device according to any one of the preceding claims, **characterized in that** a multiaxial control is provided for controlling the movement of the function head, wherein a plurality of axles is controllable simultaneously by means of one or more data input configurations, and wherein the movement characteristics of the manipulator configuration can be adjusted by transformation of the control coordinate system to any point, wherein the control point is preferably placed at a point of contact of the function head with the body.

11. The movement device according to any one of claims 1 through 10, **characterized in that** the movement device is embodied as a flight simulator, as a one-arm centrifuge, as a centrifuge having a movable slide, as a centrifuge having a movable heave slide, as a training centrifuge, as a training centrifuge for use in zero gravity, as a medical centrifuge or as a medical centrifuge having a plurality of nacelles arranged rotatably about a first axis of rotation (32).

12. The movement device according to any one of claims 1 through 11, **characterized in that** a base carrier element (35) that is coupled or connected to the first carrier element (33) is provided and is connected or coupled or can be connected to the base (4) of the manipulation configuration (1).

13. The movement device according to any one of claims 1 through 12, **characterized in that** the function head (3) is movable essentially along a tangential plane of the main axis (32) by means of the force-limited drive device (29) or by means of the force-limited drive devices (29).

14. The movement device according to any one of claims 1 through 13, **characterized in that** the function head (3) can be guided to the body (2) and/or positioned in relation to the body (2) by remote control or automation by means of a data input configuration (37) during rotation about the main axis (32) by actuation of the manipulator configuration (1).

15. The movement device according to any one of claims 1 through 14, **characterized in that** the function head (3) can be guided to the body (2), pressed against the body (2) and/or positioned on the body (2) by remote control or automation in a selectable position and/or with a selectable contact force during rotation about the main axis (32) by actuation of the manipulator configuration (1) by means of a data input configuration (37).

16. The movement device according to any one of claims 1 through 15, **characterized in that** the position and/or the contact force between the function head (3) and the body (2) can be varied by remote control or automation by actuation of the manipulator configuration (1) by means of a data input configuration (37) during rotation about the main axis (32).

17. The movement device according to any one of claims 1 through 16, **characterized in that** the contact force is selectable, and the contact force is limited by a pressure valve of the force-limited drive device, wherein the pressure valve is embodied as a regulating valve.

## Revendications

1. Dispositif de déplacement avec au moins un dispositif de fixation (31) pour recevoir, placer et/ou fixer un corps humain ou animal (2), prévu sur un premier élément de support (33) disposé en rotation autour d'un premier axe principal (32),
le dispositif de déplacement étant réalisé sous la forme d'une centrifugeuse, d'une centrifugeuse médicale ou d'une centrifugeuse d'exercice,
caractérisé
∘ en ce qu'un ensemble de manipulation (1) est disposé sur le dispositif de déplacement,
∘ en ce que l'ensemble de manipulation (1) est adapté pour un examen ou traitement tactile et/ou invasif sur un corps humain ou animal (2) sous l'influence d'une accélération accrue et/ou variable de manière permanente,
dans lequel une accélération accrue et/ou variable est un état d'accélération dans lequel l'ensemble de manipulation (1) subit des forces d'accélération accrue et/ou variable et est déplacé,
∘ en ce que l'ensemble de manipulation (1) comprend une tête fonctionnelle (3), dans lequel le déplacement et la modification du déplacement fait à l'ensemble de manipulation (1) et à la tête fonctionnelle subir des forces d'accélération qui sont différentes des forces d'accélération de l'environnement,
∘ en ce que la tête fonctionnelle (3) peut être déplacée par rapport à une base (4) selon plusieurs degrés de liberté pouvant être déplacés par des dispositifs d'entraînement, dans lequel la position et/ou la force de contact de la tête fonctionnelle (3) en contact avec le corps (2) peuvent être modifiées par rapport au corps (2) sous l'influence d'une accélération accrue et/ou variable,
∘ en ce qu'au moins un dispositif d'entraînement est réalisé sous la forme d'un dispositif d'entraînement à force limitée (29), et en ce que la force de contact est limitée de manière systémique par le dispositif d'entraînement à force limitée, de façon à exclure toute blessure accidentelle du corps par la tête fonctionnelle et/ou l'ensemble de manipulation (1), et en particulier en ce que la force de contact est limitée par une soupape de pression du dispositif d'entraînement à force limitée.

2. Dispositif de déplacement selon la revendication 1, **caractérisé en ce que** le dispositif d'entraînement à force limitée (29) comprend un ou plusieurs actionneurs pneumatiques sans piston, un ou plusieurs ensembles de muscles pneumatiques, un ou plusieurs ensembles de soufflet pneumatique, un ou plusieurs ensembles de vérins pneumatiques avec des vérins pneumatiques munis de pistons montés substantiellement sans adhérence, une ou plusieurs unités linéaires électriques sans réducteur avec des ancrages montés substantiellement sans adhérence et/ou un ou plusieurs guidages.

3. Dispositif de déplacement selon l'une quelconque des revendications 1 ou 2, **caractérisé en ce que** les dispositifs d'entraînement comprennent un axe linéaire, un entraînement par crémaillère, un entraînement cinématique parallèle, un hexapode, un tripode, un bras robotique, un entraînement rotatif, un entraînement par cardan et/ou un entraînement cartésien.

4. Dispositif de déplacement selon l'une quelconque des revendications 1 à 3, **caractérisé en ce que** les dispositifs d'entraînement comprennent respectivement un élément de base, un élément de déplacement et un entraînement pour déplacer l'élément de déplacement par rapport à l'élément de base selon le ou chaque degré de liberté, et **en ce que** les dispositifs d'entraînement sont juxtaposés en série, dans lequel respectivement l'élément de déplacement d'un dispositif d'entraînement est relié ou couplé à l'élément de base du dispositif d'entraînement suivant.

5. Dispositif de déplacement selon l'une quelconque des revendications 1 à 4, **caractérisé en ce que** les dispositifs d'entraînement sont commandés et/ou régulés par un ou plusieurs dispositifs de commande (36), et **en ce que** les dispositifs d'entraînement et les dispositifs de commande (36) sont configurés pour fonctionner sous une accélération accrue et/ou variable.

6. Dispositif de déplacement selon l'une quelconque des revendications 1 à 5, **caractérisé en ce que** la tête fonctionnelle (3) peut être rapprochée du corps (2) dans une position au choix.

7. Dispositif de déplacement selon l'une quelconque des revendications 1 à 6, **caractérisé en ce que** le déplacement de la tête fonctionnelle (3) par rapport à la base (4) peut être télécommandé et/ou automatisé, en particulier par un ou plusieurs dispositifs de commande (36) et/ou un ou plusieurs ensembles d'entrée de données (37).

8. Dispositif de déplacement selon l'une quelconque des revendications 1 à 7, **caractérisé en ce que** l'ensemble d'entrée de données (37) comprend des appareils d'entrée, tels que des manettes, des curseurs, des gants électroniques, des programmes informatiques, des programmes automatisés et/ou des ensembles similaires.

9. Dispositif de déplacement selon l'une quelconque des revendications 1 à 8, **caractérisé en ce que** la tête fonctionnelle (3) comprend des composants, des dispositifs d'examen et/ou de traitement, comme par exemple une tête de mesure à ultrasons, un appareil d'enregistrement optique, un appareil d'enregistrement acoustique, un appareil de mesure de résistance, un ensemble d'injection, un ensemble d'analyse de liquide, un dispositif de prise de sang, un dispositif d'analyse, un dispositif d'analyse chimique, une source de rayons, par exemple de rayons X, gamma ou infrarouges, une source laser, un dispositif d'échantillonnage, un appareil de mesure de température, un ampèremètre, un dispositif de détection de rayons, un appareil d'examen par endoscopie, un dispositif pour un examen ophtalmologique optique et/ou d'autres dispositifs radiologiques invasifs ou tactiles à vocation diagnostique ou thérapeutique.

10. Dispositif de déplacement selon l'une quelconque des revendications précédentes, **caractérisé en ce que** pour la commande du déplacement de la tête fonctionnelle, une commande sur plusieurs axes est prévue avec laquelle un ou plusieurs ensembles d'entrée de données permettent de commander plusieurs axes en même temps, et dans lequel la caractéristique de déplacement de l'ensemble de manipulation peut être adapté par la transformation du système de coordonnées de commande à un point quelconque , le point de commande étant fixé de préférence en un point de contact de la tête fonctionnelle et le corps.

11. Dispositif de déplacement selon l'une quelconque des revendications 1 à 10, **caractérisé en ce que** le dispositif de déplacement est réalisé sous la forme d'un simulateur de vol, d'une centrifugeuse à bras unique, de centrifugeuse à chariot mobile, de centrifugeuse à chariot de levage mobile, de centrifugeuse d'exercice, de centrifugeuse d'exercice pour la mise en œuvre en apesanteur, de centrifugeuse médicale ou de centrifugeuse médicale avec plusieurs nacelles disposées en rotation autour d'un premier axe de rotation (32).

12. Dispositif de déplacement selon l'une quelconque des revendications 1 à 11, **caractérisé en ce qu'**un élément de support de base (35) couplé ou relié au premier élément de support (33) est prévu qui est relié, couplé ou peut être relié à la base (4) de l'ensemble de manipulation (1).

13. Dispositif de déplacement selon l'une quelconque des revendications 1 à 12, **caractérisé en ce que** la tête fonctionnelle (3) peut être déplacée par le dispositif d'entraînement à force limitée (29) ou par les dispositifs d'entraînement à force limitée (29) substantiellement le long d'un plan tangent de l'axe principal (32).

14. Dispositif de déplacement selon l'une quelconque des revendications 1 à 13, **caractérisé en ce que** pendant la rotation autour de l'axe principal (32), la tête fonctionnelle (3) peut être guidée vers le corps (2) et/ou positionnée par rapport au corps de manière télécommandée ou automatisée par l'actionnement de l'ensemble de manipulation (1) par un ensemble d'entrée de données (37).

15. Dispositif de déplacement selon l'une quelconque des revendications 1 à 14, **caractérisé en ce que** pendant la rotation autour de l'axe principal (32), la tête fonctionnelle (3) peut être guidée et appliquée au corps (2) et/ou positionnée par rapport au corps de manière télécommandée ou automatisée dans une position au choix et/ou avec une force de contact au choix par l'actionnement de l'ensemble de manipulation (1) par un ensemble d'entrée de données (37).

16. Dispositif de déplacement selon l'une quelconque des revendications 1 à 15, **caractérisé en ce que** pendant la rotation autour de l'axe principal (32), la position et/ou la force de contact entre la tête fonctionnelle (3) et le corps (2) peuvent être modifiées de manière télécommandée ou automatisée par l'actionnement de l'ensemble de manipulation (1) par un ensemble d'entrée de données (37).

17. Dispositif de déplacement selon l'une quelconque des revendications 1 à 16, **caractérisé en ce que** la force de contact peut être sélectionnée, et **en ce que** la force de contact est limitée par une soupape de pression du dispositif d'entraînement à force limitée, la soupape de pression étant réalisée sous la forme d'une soupape de réglage.
